(19) Europäisches Patentamt / European Patent Office / Office européen des brevets

(11) **EP 3 902 453 B1**

(12) **EUROPEAN PATENT SPECIFICATION**

(45) Date of publication and mention
of the grant of the patent:
**29.01.2025 Bulletin 2025/05**

(21) Application number: **19905070.9**

(22) Date of filing: **26.12.2019**

(51) International Patent Classification (IPC):
*A47J 37/12* (2006.01)   *A23D 9/00* (2006.01)
*A23L 5/10* (2016.01)   *G01N 27/20* (2006.01)
*G01N 27/22* (2006.01)   *G01N 21/359* (2014.01)
*G01N 33/03* (2006.01)   *G01N 21/3577* (2014.01)

(52) Cooperative Patent Classification (CPC):
**C11B 3/008; A23D 9/04; A23L 5/11;**
**G01N 21/3577; G01N 21/359;** A47J 37/1271;
G01N 27/22; G01N 33/03

(86) International application number:
**PCT/US2019/068587**

(87) International publication number:
**WO 2020/139952 (02.07.2020 Gazette 2020/27)**

(54) **EDIBLE OIL MANAGEMENT INCLUDING SENSING AND MODELING**

VERWALTUNG VON SPEISEÖL MIT ERFASSUNG UND MODELLIERUNG

GESTION D'HUILE COMESTIBLE COMPRENANT LA DÉTECTION ET LA MODÉLISATION

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **27.12.2018 US 201862785441 P**

(43) Date of publication of application:
**03.11.2021 Bulletin 2021/44**

(73) Proprietor: **Cargill, Incorporated**
**Wayzata, Minnesota 55391 (US)**

(72) Inventors:
• **IASSONOVA, Diliara**
**Maple Grove, Minnesota 5311 (US)**
• **DEBONTE, Lorin Roger**
**Eden Prairie, Minnesota 55347 (US)**

(74) Representative: **Elseviers, Myriam**
**Cargill R&D Centre Europe BVBA**
**Bedrijvenlaan 9**
**2800 Mechelen (BE)**

(56) References cited:
EP-A1- 1 004 872   WO-A1-2015/142283
WO-A1-2017/002079   JP-B2- 5 303 693
US-A1- 2009 252 842   US-A1- 2011 129 578
US-A1- 2015 027 205   US-A1- 2018 116 459
US-A1- 2018 125 299   US-B2- 7 885 521

**Description**

FIELD OF THE DISCLOSURE

This document pertains generally, but not by way of limitation, to management of edible oil, and more particularly, to systems and techniques for determination of edible oil quality.

BACKGROUND

Food scientists may employ a variety of analytical tools to assist in quantitative evaluation of various characteristics of products, from raw materials to finished goods. Generally, analytical tools may rely upon careful control and preparation of a sample for evaluation, such as according to a standardized test or evaluation protocol in a "bench" setting. In this manner, traceable and repeatable results may be obtained. Examples of such techniques, such as may be applied to edible oils, include Fourier Transform (FT) Infrared (IR) Spectroscopy, Nuclear Magnetic Resonance (NMR), Gel Permeation Chromatography (GPC), and Gas Liquid Chromatography (GLC), as illustrative examples. Use of analytical techniques to evaluate edible oils helps to verify or maintain quality throughout the production and distribution process. For example, after processing, bench analytical techniques may be used to verify that minor components, such as free fatty acid, are at or below specified levels. Analytical techniques may also be used to assess edible oils for a presence of contaminants or adulterants. Edible oils used for frying food, such as in deep fat frying applications, undergo a variety of degradation mechanisms as such oils age in use. Such degradation mechanisms may adversely impact oil quality, and may include oxidative processes, hydrolysis, and pyrolysis, as illustrative examples.

Patent document WO 2017/002079 A1 discloses a device and a method for real-time measurement of the quality of frying oil by sensing a chemical species related to the quality of the frying oil. This device is useful for measuring the frying oil parameters so the user may know when the oil organoleptic properties are no longer adequate for consumption or use. Said device comprises an optical sensor, a chamber for receiving the frying oil to be measured, an analogue signal processing module, a transmitting unit, a receiver, a processing unit, a display device and an integrated information system.

SUMMARY OF THE DISCLOSURE

Various "open loop" schemes may be used by frying equipment operators or service providers to carry out management of frying oil. Such management might include logistics relating to procurement and storage of fresh oil stock, dispensing of fresh oil into individual frying stations, filtering of frying oil after some duration of use, and removal of oil from use, including management of related storage and disposal. In the simplest scheme, such management might include ordering a fixed volume of replacement oil on a periodic basis, and initiating operations such as fryer cleaning, oil filtering, or oil replacement based exclusively on attributes such as absolute oil age, duration of use at frying temperature, or a count of "drops" of a frying basket. Top-off may be initiated in response to volumetric loss of oil over a duration of frying drops.

Oils used for frying may also be managed (e.g., filtered or replaced) subject to a "go/no-go" end-use monitoring regime. Such monitoring may facilitate compliance with regulatory requirements, and such requirements may be specific to particular geographic locales. Such requirements may be specified in terms of limits relating to polar material (e.g., Total Polar Material (TPM)), free fatty acid (FFA) content, color (e.g., Gardner color unit), or polymer content, as illustrative examples.

The present inventors have recognized that open-loop schemes or "go/no-go" monitoring as mentioned above may present various challenges, such as leading to inconsistent frying quality, or inefficient utilization or management of edible oil inventory. For example, an open-loop scheme may result in unnecessary waste where oil replacement is initiated more frequently than is necessary given regulatory or food-quality constraints (e.g., an overly-conservative scheme). In another example, an open-loop scheme may result in inconsistency because the edible oil goes through a wider progression of the degradation spectrum spanning a range of quality from new (e.g., unused) to well beyond an optimum state (e.g., at or near replacement).

The present inventors have also recognized an unexpected result that fresh (e.g., unused oil) does not necessarily produce fried food having the highest perceived quality, when such food is evaluated according to more subjective factors such as flavor, aroma, texture (e.g., mouth-feel), or the like. To address various challenges mentioned above, the present inventors have developed an analytical model representing a "composite" aging parameter (e.g., a quality index indicative of aging). The analytical model may be defined as a function of various attributes, such as accumulated frying hours compensated for by top-offs. The model can include a mass balance determination of aged oil starting from fresh oil and including top-offs. Other parameters in the analytical model include factors relating to sensed oil quality, or other attribute information. The model is used predictively, such as combined with historical data to predict oil consumption based on historical oil consumption metrics coupled with modeling of oil aging. An aging parameter provided by the model may be used to trigger action such as notification to perform actions, or such a parameter may be used to automate one or more actions relating to oil management in frying operations. An analytically-modeled aging parameter may also be used prognostically to predict aging based on one or more of sensed data from a fryer or attribute data relating to frying operations.

Generally, the present inventors have recognized that a higher or highest perceived quality of fried food may fall within an intermediate range of the aging parameter provided by the model. Such a model may be used to calculate a running composite aging value (e.g., a quality index), and an equilibrium may exist as a function of cumulative frying time and top-offs, or as a function of other attributes. Accordingly, an equilibrium may be targeted to maintain the edible oil in the intermediate age range, such as by triggering one or more of partial top-offs with fresh oil, or partial removal of oil from a frying station reservoir, as illustrative examples. In a passive monitoring scenario, the model may be used to report an oil age predictively for use in planning future top-offs, removal of oil, filtration, or oil replacement. In this manner, a technical solution is presented that may be used to maintain edible oil in a specified quality index range in frying operations or predicting an oil consumption metric (e.g., anticipated oil volume per unit time, volume of oil remaining at a future time, an indication of a volume of oil recommended for use in top-offs at a future time or a over a specified duration) to aid in forecasting or inventory management.

The present inventors have also recognized, among other things, that testing, logging, and analyzing parameters relating to edible oils in actual use scenarios may present various challenges. For example, bench analytical techniques may provide robust repeatability for evaluation of samples, but sample preparation may be cumbersome and may not generally provide results in real-time or near-real-time. Moreover, use of bench analytical techniques may involve specialized skills in sample preparation, sample handling, execution of tools for quantitative analysis, and reporting of results. Even if the sampling process were simplified, unskilled personnel may have difficulty in interpreting results reported quantitatively in terms of TPM, FFA, a color metric, a polymer content, or other technical parameters. Challenges may also exist in relating analytically-measured parameters such as TPM, FFA, color, or polymer to more subjective measures of food quality. Accordingly, the present inventors have also recognized that one or more sensors can be interfaced with or even mechanically integrated within a frying apparatus to facilitate implementation of an automated or semi-automated oil management scheme.

In an example, one or more of top-off, partial or complete drainage, or filtering operations may be entirely automated and triggered in response to determined quality index values or predicted quality index values provided by applying the analytical model. The analytical model may also include or may be combined with data obtained using one or more sensors incorporated in the frying apparatus. Such sensors may include operational parameters such as temperature, duration at temperature, a count of "drops," or analytical measurements. For example, an optical sensing scheme may be used to provide data indicative of one or more of TPM, FFA, a color metric, a polymer content, or other technical parameters. The analytical model may include weighting factors corresponding to one or more such technical parameters to enhance monitoring or predictive accuracy. As an illustrative example, the monitoring schemes described herein may include in-situ use of one or more of reflectance or transmission spectroscopy, such as in a near-infrared (NIR) range of optical wavelengths.

In an example, a technique, such as a machine-implemented method, can include monitoring a characteristic of edible oil using an optical technique, to manage oil quality in frying operations. The technique can include obtaining a sensed characteristic of edible oil in a frying apparatus, using an optical sensor, receiving data indicative of an attribute other than obtained using the optical sensor, automatically determining an edible oil quality index using an analytical model, the analytical model defined as a function of the sensed characteristic and the attribute, and, in response to determining the edible oil quality index, automatically triggering an action.

In an example, a technique, such as a machine-implemented method, can include predicting a characteristic of edible oil to manage oil quality in frying operations. The technique can include obtaining a sensed characteristic of edible oil in a frying apparatus using a sensor, the sensed characteristic indicative of edible oil degradation, receiving data indicative of an attribute other than obtained using the sensor, the attribute defining a future operational condition for frying operations, automatically determining a predicted edible oil quality index using an analytical model, the analytical model defined as a function of the sensed characteristic and the attribute, and in response to determining the predicted edible oil quality index, triggering an action.

In an example, a system can provide oil quality management, the system comprising a frying oil reservoir containing edible oil, a sensor to obtain a sensed characteristic indicative of degradation of the edible oil, a controller configured to receive data indicative of an attribute other than obtained using the sensor, along with the sensed characteristic, the controller configured to determine a predicted edible oil quality index using an analytical model, the analytical model defined as a function of the sensed characteristic and the attribute, and in response to determining the predicted edible oil quality index, the controller triggering an action including one or more of top-off of the edible oil, removal of at least a portion of the edible oil, filtration of the edible oil, or replacement of the edible oil.

In an example, a technique, such as a machine-implemented method, can include receiving data indicative of an attribute of edible oil used in frying apparatus, automatically applying an analytical model indicative of an oil quality index to provide a predicted oil consumption metric, the analytical model defined as a function of a sensed characteristic and the attribute, in response to applying the analytical model, triggering an action based on a predicted oil consumption metric.

Generally, according to various examples, a characteristic of edible oil used for frying operations may be determined using a sensor such as a spectrometer or capacitive sensor. Other attributes indicative of a prior, present, or future frying operations can be specified, and an analytical model indicative of a composite aging parameter can be applied using one or more attributes along with data obtained from the sensor indicative of oil degradation. Application of the analytical model may be used to trigger various actions relating to oil management, such as ranging from automatically generating a notification to an operator or automatically initiating oil management operations such as top-off, oil removal, or oil replacement in a frying apparatus.

The current invention provides a method according to attached claim 1. Further embodiments are disclosed in the dependent claims.

This summary is intended to provide an overview of subject matter of the present patent application. It is not intended to provide an exclusive or exhaustive explanation of the invention. The detailed description is included to provide further information about the present patent application.

BRIEF DESCRIPTION OF THE DRAWINGS

In the drawings, which are not necessarily drawn to scale, like numerals may describe similar components in different views. Like numerals having different letter suffixes may represent different instances of similar components. The drawings illustrate generally, by way of example, but not by way of limitation, various embodiments discussed in the present document.

FIG. 1 illustrates generally an example showing a system that can include an oil reservoir such as a vat in a frying apparatus.

FIG. 2 illustrates generally a modeling scheme that can be used to provide a quality index indicative of edible oil aging, such as for use in managing edible oil used in frying operations.

FIG. 3 illustrates generally an example of a continuum of edible oil management schemes that can be supported by a modeling scheme as described in relation to various other examples.

FIG. 4 illustrates generally a technique, such as a method, including use of an edible oil quality index to trigger actions in relation to managing edible oil used in frying operations.

FIG. 5 illustrates generally a technique, such as a method, including use of an edible oil quality index to automatically control operations relating to managing edible oil used in frying operations.

FIG. 6 illustrates generally a technique, such as a method, including use of an edible oil quality index to automatically control operations relating to managing edible oil used in frying operations, including automatically modifying control of edible oil operations.

FIG. 7 illustrates generally a technique, such as a method, including use of surrogate data from a sensor to determine a parameter (e.g., an attribute) for use in determining an edible oil quality index.

FIG. 8 illustrates generally an example comprising a multi-loop control scheme such as for use in managing edible oil used in frying operations.

FIG. 9A illustrates generally a range of variation in various characteristics indicative of edible oil degradation, such as corresponding to an open-loop or go/no-go based monitoring scheme.

FIG. 9B illustrates generally a reduced range of variation in various characteristics indicative of edible oil degradation, such as targets that may be achieved using an edible oil quality index to control operations relating to managing edible oil used in frying operations.

FIG. 9C illustrates generally a comparison between a running cumulative total of oil volume consumed using an open-loop or go/no-go monitoring scheme, as compared to a using an edible oil quality index to control operations relating to managing edible oil used in frying operations.

FIG. 10A illustrates generally experimentally-obtained measurements of Total Polar Material (TPM) percentages corresponding to three different frying oils over the course of a study, according to an illustrative example.

FIG. 10B illustrates generally experimentally-obtained measurements of Free Fatty Acid (FFA) percentages corresponding to the three different frying oils over the course of the study, according to an illustrative example.

FIG. 10C illustrates generally experimentally-obtained measurements of a color metric (e.g., Gardner Red color unit) corresponding to the three different frying oils over the course of the study, according to an illustrative example.

FIG. 10D illustrates generally an oil quality index represented as an equivalent age in days corresponding to the three different frying oils over the course of the study, according to an illustrative example.

FIG. 11 illustrates a block diagram of an example comprising a machine upon which any one or more of the techniques (e.g., methodologies) discussed herein may be performed.

DETAILED DESCRIPTION

As mentioned above, the present inventors have recognized that a combination of sensed information and an analytical model can be used in the management of edible oil for use in a frying apparatus. Use of such a model can facilitate monitoring or prediction of oil aging and can help to maintain food quality by controlling an edible oil quality index, indicative of edible oil aging, to seek or maintain an equilibrium in a controlled quality index range to provide predictable food quality. For example, control of a quality index range can encompass targeting an intermediate range of oil age determined using the model. Maintenance of the equilibrium can be accomplished using one or more sensors. Various operations such as oil top-off, replacement, or filtration can be triggered automatically. Monitored or predicted oil aging parameters can be stored and retrieved, such as along with other sensed information, to aid in refinement of model parameters, or to aid in logistical analysis such as replenishment or forecasting. The model may be used predictively, such as having inputs modified to reflect changes in various attributes such as relating to oil, fryer, food, location (e.g., store/restaurant) or operator attributes.

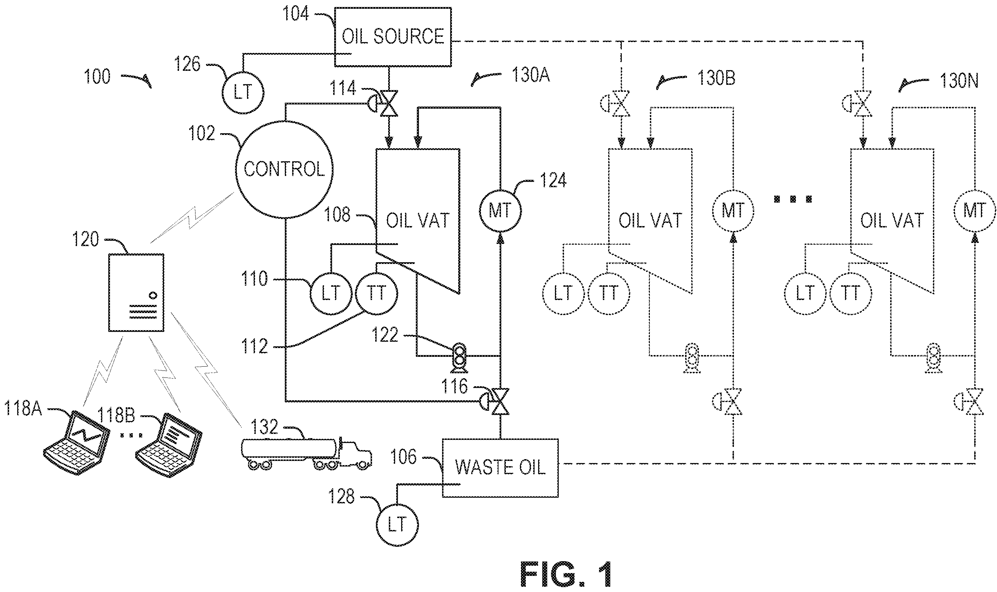

FIG. 1 illustrates generally an example showing a system 100 that may include an oil reservoir such as an oil vat 108 in a frying apparatus 130A. The controller 102 may be shared across one or more fryers such as shown as frying apparatus 130B through frying apparatus 130N, or each frying apparatus 130A, 130B, through 130N may be communicatively coupled to a respective dedicated controller 102. Various elements of the system 100 may be communicatively coupled to each other, such as via a wired or wireless link. Edible oil in use in the frying apparatus 130A may be monitored using an oil temperature sensor 112 (e.g., a temperature transmitter) or an oil level sensor 110 (e.g., a level transmitter). The oil temperature sensor 112 and the oil level sensor 110 may be communicatively coupled to a controller 102, such as to regulate one or more of an oil level or an oil temperature associated with the oil vat 108. As mentioned elsewhere herein, one or more other characteristics of an edible oil used in the frying apparatus 130A may be monitored. For example, in one approach, go/no-go monitoring may be performed using chemical test strips or a hand-held electronic testing device, such as to provide an indication of one or more oil characteristics indicative of oil degradation, such as a Free Fatty Acid (FFA) value, a Total Polar Material (TPM) value, or a color metric, as illustrative examples. Such test strips or the hand-held electronic device can provide a value that can be manually compared with a limit, and edible oil located in the oil vat 108 can be filtered or replaced based on such a comparison.

By contrast, the system 100 shown in FIG. 1 can provide a semi-automated or fully-automated management scheme for edible oil used by the frying apparatus 130A. For example, the system 100 may include a sensor such as an optical sensor or electrical sensor (e.g., a measurement transmitter 124) communicatively coupled with the controller 102 or other portions of the system 100, such as to provide data indicative of a characteristic of the edible oil used by the frying apparatus 130A to the controller 102. As illustrative examples, the measurement transmitter 124 may include an optical sensor configured to perform at least one of an optical transmission measurement through the edible oil or an optical reflectance measurement to obtain a reflection from a surface of the edible oil, such as for performing a spectroscopic measurement. In another example, the measurement transmitter may include an electrical or impedance sensor, such as a capacitive sensor.

As an example, spectroscopic evaluation using the measurement transmitter 124 can include obtaining measurements in an infra-red range of wavelengths, such as to obtain a series of values corresponding to discrete wavelengths spanning a specified range of wavelengths. As an illustrative example, the specified range may include a near-infrared range of wavelengths from about 700 nanometers to about 1100 nanometers. The controller 102 or other portion of the system 100 may extract a specified characteristic from measurement provided by the optical sensor, such as using a regression technique relating the obtained transmission or reflectance values to an FFA value, a TPM value, a color metric, or a polymer content, as illustrative examples. As shown in FIG. 1, the measurement transmitter 124 may be in fluidic communication with the oil vat 108, such as through a measurement loop fed by a circulating pump 122. Other configurations may be used, such as including the measurement transmitter 124 as a portion of a filtration loop, or placing the measurement transmitter 124 in a location to observe oil in the oil vat 108 directly. In yet another example, the measurement transmitter 124 may include a compact (e.g., hand-held) housing and hardware configuration. For example, the SCiO apparatus (available from Consumer Physics, Tel Aviv, Israel), may be used to obtain optical measurements of the edible oil in communication with the controller 102 or other portions of the system 100, such as to obtain reflectance data across a specified range of wavelengths (e.g., a range of 750 nanometers to 1070 nanometers, for example). The use of reflectance spectroscopy in the near-infrared range of wavelengths is illustrative, and other spectroscopic or electrical measurement techniques may be used.

The controller 102 may implement an oil-management scheme such as using information obtained from the measurement transmitter 124, along with one or more other attributes (such as oil level, oil temperature, trends in oil level or temperature, or attribute information concerning future operations). The controller 102 may instantiate an analytical model defining an oil quality index, such as for use in monitoring degradation of the edible oil in the oil vat 108 or for predicting a progression of such degradation. The controller 102 may be communicatively coupled to other portions of the system 100 such as a data processing system 120 (e.g., an on-site server or site-wide control system, or a remote system such as a cloud-based management system, as illustrative examples). The data processing system 120 may be used to receive attribute information concerning historical, present, or future operations. The controller 102 may apply the analytical model and a resulting oil quality value or a predicted oil quality value may be used to trigger one or more actions, or the controller 102 may communicate with the data processing system 120 to trigger application of the model by the data processing system 120 or to receive commands generated by the data processing system 120 in view of model evaluation results. As described in relation to other examples herein, a triggered action may include notifying an operator to perform an oil top-off, a filtration, a removal of oil, or replacement of oil. In another example, such as shown illustratively in FIG. 1, the controller 102 may be communicatively coupled to one or more valves, such as fresh oil supply valve 114 or an oil drain valve 116. The controller 102 may open one or more of the oil supply valve 114 or the oil drain valve 116 on a controlled basis, such as in response to present or predicted oil quality index values as provided by the analytical model.

An oil source 104 such as a bulk fresh oil reservoir may be monitored by a level sensor 126. Similarly, a waste oil 106 level may be monitored by a level sensor 128. One or more of the level sensor 126 or the level sensor 128 may be communicatively coupled to the controller 102 or the data processing system 120. In response to one or more of sensed level information, present oil-quality index value, or predicted future oil-quality index values, the data processing system 120 or other portions of the system 100 may trigger replenishment of the oil source 104, disposal of waste oil 106, or both. For example, a request or dispatch of fleet 132 oil delivery or disposal may be triggered in response to such monitoring or prediction. As an illustrative example, if a present or predicted quality index value indicated by the analytical model correlates with higher oil consumption, the data processing system 120 may generate or modify forecasting of fresh oil demand or waste oil generation for use in planning or dispatch of fleet oil supply or disposal resources. In an example, historical data such as indicative of time of day, time of month, time of year, holidays or times relative to holidays, or seasonal information can be provided as attribute information to the model, or such information can be logged in relation to other indicia of oil degradation for use in future application of the model, such as to provide predictive capability. As an illustrative example, an analytical model can be used to monitor or predict oil degradation across various oil volume demand scenarios, such as to assist with forecasting or generate notifications or requests to aid in capacity planning or inventory management for frying operations.

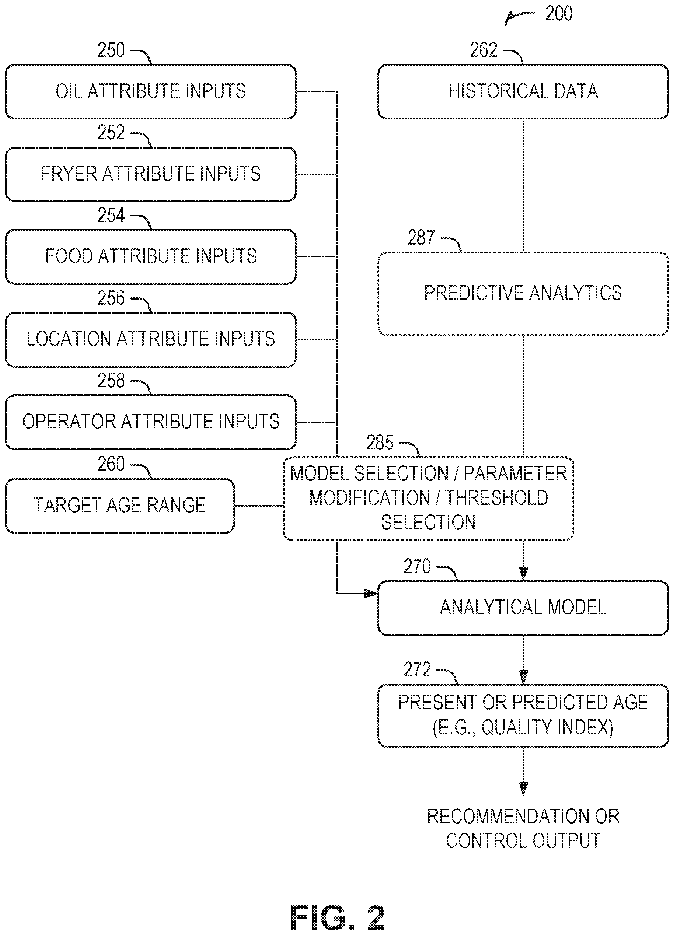

Attribute information other than sensed information provided by the measurement transmitter 124 may be stored in the controller 102, such as received from one or more operator interfaces (e.g., client machines 118A or 118B, such as computers, tablets, laptops, or fixed operator terminals having a display and input device). As discussed below in FIG. 2, attribute information may include specific attribute data concerning the oil (e.g., temperature, volume), the frying apparatus 130A, food attributes, store attributes, or operator attributes. The data processing system 120 may implement an expanded instantiation of the analytical model, and the controller 102 may be configured to implement a simplified instantiation of the analytical model for use in management of edible oil in the frying apparatus 130A. According to various examples described in this document, the controller 102 may trigger actions involving operator intervention (e.g., notifications to one or more of add oil, remove oil, filter oil, or replace oil, or inhibit (e.g., lockout) frying apparatus from use), or the controller 102 may automatically perform one or more actions. In an example, as described elsewhere herein, the controller 102 may modify one or more operational conditions such as to maintain the oil quality index in a specified range.

FIG. 2 illustrates generally a modeling scheme 200 that can be used to provide a quality index indicative of edible oil aging, such as for use in managing edible oil used in frying operations. As mentioned in relation to other examples herein, present or predicted values of the oil quality index 272 may be used to automatically control at least some oil management operations relating to a frying apparatus or to furnish recommendations regarding such management, such values may be determined by application of an analytical model 270. The present inventors have recognized, among other things, that an analytical model 270 can represent a "composite" edible oil aging parameter (e.g., a quality index 272 indicative of aging), such as defined as a function of various attributes. Such an analytical model 270 may also be used prognostic ally to predict edible oil aging based on sensed data from a fryer and attribute data relating to frying operations.

The quality index 272 provided by the analytical model can be compared to one or more thresholds, such as to trigger one or more respective operations such as oil top-off, oil filtration, oil removal, or oil replacement. In an example, oil top-off operations or removal operations can be performed on a recurrent basis, such as initiated or modified to constrain the quality index 272 within a specified range of values. For example, the present inventors have recognized that a higher or highest perceived quality of fried food may fall within an intermediate range of the quality index 272 provided by the model 270, and an equilibrium may exist as a function of cumulative frying time and top-offs, or as a function of other attributes.

Attribute inputs provided to the model may be selected or constrained to various categories. For example, as shown in FIG. 2, oil attribute inputs 250 may include one or more of type of oil, sensed oil temperature, sensed oil color (e.g., analyzed according to a color metric), sensed TPM or other data indicative of polar molecule content, FFA, aldehyde content, oil volume, or a trend of any of the previous attributes evaluated over time or in terms of duration (e.g., duration at temperature, color or other parameter vs. time, change in oil volume vs. time). Fryer attribute inputs 252 may include one or more of a count of basket drops (or a count of events serving as a surrogate for basket drops, as mentioned elsewhere herein), a count of filtration operations, a cleaning history (e.g., a count of cleaning events), or a trend of any of the previous attributes evaluated over time or in terms of duration (e.g., duration since last cleaning event, total count of cleaning events over a specified timeframe, basket drops per unit time, or the like). Food attribute inputs 254 may include one or more attributes relating to the prior or intended use of the frying apparatus, including an oil take-up rate or oil take-up volume per basket drop or per unit time, a holding time prior to frying or after frying, a type of food (potatoes versus chicken), or an indication whether the frying operation will be par-frying, as illustrative examples. Location attribute inputs 256 may include one or more location attributes such as specified in terms of geographic location or temporal attributes, such as time of day, time of week, time of month, time of year, or in terms of operational volume per unit time (e.g., basket drops per unit time, such as specified for a particular range of times, such as by time of day, time of week, time of month, time of year, etc.).

Operator attribute inputs 258 may include one or more operator attributes such as specified in terms of particular operator characteristics (including scaling or adjusting any of the other parameters in a manner specific to a particular vendor, or establishing vendor-specific or geography-specific thresholds or limits). Historical data 262 representative of any of the attribute inputs 250, 252, 254, 256, or 258 may also be used to establish one or more of the analytical model coefficients, parameters to be included, thresholds for control, or even to select which model to use. As an illustrative example, a quality index threshold or a desired range of quality index values (e.g., a target age range 260) may be established using empirical techniques for a range of different attribute inputs or other conditions. Model selection or parameter selection can be made based on historical information such as a time of year, a time of month, a time of day, weekend vs. weekday, holiday operation, non-holiday operation, such as to enhance predictive performance when oil consumption or degradation would correlate with a predicted customer "rush." The modeling scheme 200 can include combining the analytical model (e.g., an oil quality index or aging metric) with predictive analytics 287, such as to provide the present or predicted quality index 272 or to provide other data such as indicative of oil consumption. For example, the analytical model 270 can be combined with predicted oil consumption data (e.g., provided by predictive analytics 287) extracted from historical data 262. Such predictive analytics 287 can be used to provide oil consumption by mass or volume per unit time given temporal data such as mentioned above (e.g., time of year, holiday / non-holiday). Such predictive analytics can be combined with the analytical model 270 such as to provide oil aging prediction in view of historical data 262, along with other attributes 250, 252, 254, 256, or 258. As an illustrative example, the analytical model 270 can be used to increase or decrease a predicted oil consumption metric based on sensed information indicative of oil aging along with other attribute information.

Model selection, parameter modification, or threshold selection 285 may be performed to tailor the application or instantiation of the analytical model 270 to a specific use case, such as based on one or more attribute inputs 250, 252, 254, 256, or 258 relating to present operations or anticipated future conditions. For example, model parameters such as weighting factors for individual parameters or the analytical model as a whole may be selected or provided on a basis specific to one or more of the attribute inputs 250, 252, 254, 256, or 258. For example, a specific instance of the analytical model may be selected or calibrated in response to a use case or "profile" defining attributes such as oil type, fryer type, fryer operating temperature, food type, location, and operator.

As an example, data can be received indicative of an attribute (e.g., amongst attributes 250, 252, 254, 256, or 258). For example, the attribute could be a temporal attribute such as time-of-day, time-of-year, holiday, eve of a holiday, or the like, as mentioned above. The model can be used to provide an estimate or a range of estimates relating to oil consumption based on comparing the attribute information with historical data to either select model parameters or select a model instance that corresponds with the present or anticipated operational state. The analytical model can also incorporate either an attribute or a sensed characteristic from the frying apparatus indicative of a present operational state. Application of the analytical model can then be used to forecast oil quality or oil consumption (when oil is maintained within a specified quality range according to the analytical model). In this manner, the analytical model can provide historical context to aid in forecast future oil quality or consumption by combining the historical context with, for example, sensed information obtained from present operations.

Below is an illustrative example of an analytical model that can be used to provide a quality index indicative of oil aging, using an initial oil volume, a recurring top-off volume, and a cumulative frying hour duration. In this illustrative example, assume that 50 pounds (lbs.) of fresh oil is the initial condition. A daily top-off of oil in pounds can be represented as "$X$," such that a daily yield, "$Y$" can be represented as $(50-X)/50$. A mass balance for any given day can be represented as $50*Y^i$, where the day is indicated by an index, "$i$." If the frying time per day is represented as "$H$" hours, then the cumulative frying hours for at a given day can be represented as $H*i$, and the mass balance multiplied by the frying hours, starting from 50 lbs. at any given day, $i$, can be represented by $(50*Y^i)*(H*i)$. Similarly, the oil weight times age corresponding to a top-off operation can be represented by $X*Y^{(i-1)}*H*(i-1)$. Accordingly, a composite quality index taking into account a 50 lb. initial condition and all top offs can be represented as:

$$F = \frac{50 * Y^i * H * i + \sum_{2}^{i}\left(X * Y^{i-1} * H * (i-1)\right)}{50}$$

which represents a function of inputs comprising top-off volume, frying time, and the index $i$, corresponding to days elapsed since the initial condition. More generically, assuming that the initial fresh oil mass may be represented by "M" pounds, and using the same notation as above for the other parameters, the quality index can be expressed as follows,

$$F(i) = \frac{M \cdot \sum_{k=1}^{i} H_i \cdot \prod_{k=1}^{i} Y_k + \sum_{j=2}^{i}\left(X_j \sum_{k=2}^{i} H_i \cdot \prod_{k=2}^{i} Y_k\right)}{M} =$$

$$\sum_{k=1}^{i} H_i \cdot \prod_{k=1}^{i}\left(1 - \frac{X_k}{M}\right) + \sum_{j=2}^{i}\left(\frac{X_j}{M} \sum_{k=2}^{i} H_i \cdot \prod_{k=2}^{i}\left(1 - \frac{X_k}{M}\right)\right)$$

The index can be iterated to provide a predicted future quality index, such as by advancing the time index and plugging in a value obtained from a previous iteration, where the quality index for a future time index ($i+1$) is expressed in terms of the prior index, $F_i$ corresponding to the age of the starting oil mass, $M$, along with a frying duration $H_i$, and a top-off mass, $X_i$:

$$F_{i+1} = \left(F_i + H_i\right) \cdot \left(1 - X_i / M\right)$$

If $F_{i+1}$ is evaluated for various combinations of a parameters, certain conditions may provide a compositional equilibrium where the quality index remains stable or changes only very slightly over extended duration. As a numerical example, if 20 pounds of oil are provided as a top-off mass, and the daily frying duration is 13.5 hours, the expression above shows that an equilibrium is approached in the range of about 4 to about 6 days after starting with fresh oil. In the example above, the quality index magnitude (e.g., value) correlates with predicted oil age. In this sense, higher values indicate greater oil aging. As mentioned elsewhere herein, a particular intermediate value does not necessarily indicate that the oil is unsuitable for use (or is predicted to be so), and such intermediate aging values may actually represent a desired operating range for frying operations based on qualitative factors such as aroma, texture, or taste, for example.

The analytical model mentioned in relation to various examples herein can (but need not) be instantiated literally as an equation, parameters, and coefficients to be calculated "on the fly." In another example, such a model may include a tabular representation, such as a multi-dimensional look up table wherein oil quality index values may be retrieved based on inputs comprising sensed data and data indicative of one or more attributes. If a look-up table (LUT) or other similar scheme is used, the model can be applied by retrieving a table value corresponding to a combination of inputs as indices to a particular table entry, or interpolation can be performed between discrete quality index values, as an illustrative example. Similarly, results from the look-up table or inputs defining indices for retrieval from the look-up table can be scaled or weighted.

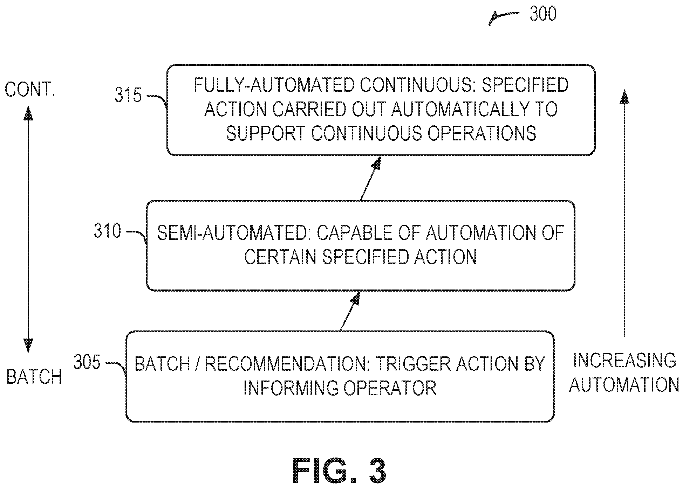

FIG. 3 illustrates generally an example of a continuum 300 of edible oil management schemes that can be supported by a modeling scheme as described in relation to various other examples described in this document. The continuum shown in FIG. 3 ranges from a batch scenario 305 to a continuous automated process at 315. At 305, for example, data obtained from a sensor (such as an optical or capacitive sensor) can be combined with attribute information such as one or more other attributes as mentioned above. An analytical model may be used to determine (e.g., numerically determine or otherwise provide) an oil quality index representative of the present state of the oil in a frying apparatus. If the evaluated oil quality index exceeds a threshold or is outside a specified range, a notification may be generated. Depending on other attribute inputs, such as a duration since the oil was replaced or since the last filtration operation, or a count of basket drops, for example, the notification may include a recommendation to replace the oil, or to perform one or more of a top-off or a removal of at least a portion of the oil. If the analytical model is used predictively, the notification may be to perform one or more of filtration, replacement, top-off, or removal of a portion of the oil to maintain the frying apparatus within the desired range of quality values (e.g., to maintain or target a desired future state). At 310, portions of the oil management scheme may be performed in an automated or semi-automated manner, such as involving recurring top-off or removal operations. In the scheme of 310, may still be batch-oriented and may be manually initiated (e.g., involving operator interaction), such as oil replacement.

The scheme in 310 may involve application of the analytical model to determine present or future quality index values, and a notification may be generated to notify the operator to modify one or more operational parameters such as a volume or frequency of top-off operations, as an illustrative example. At 315, a fully automated scheme may involve one or more of automated top-off, removal of oil, filtration, or replacement, such as without requiring operator intervention. In the scheme at 315, an interval between oil replacement operations may be extended by using a closed-loop scheme where one or more of oil top-off, filtration, or oil removal operations are adjusted to maintain the quality index below a desired threshold or within a desired range.

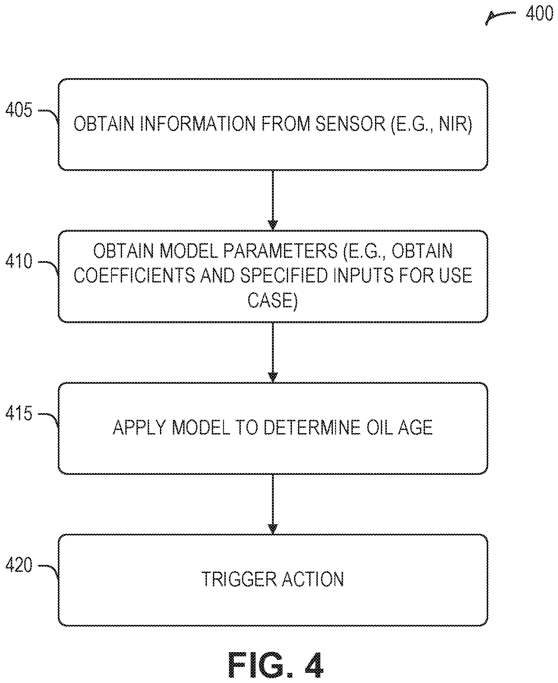

FIG. 4 illustrates generally a technique 400, such as a method, including use of an edible oil quality index to trigger actions in relation to managing edible oil used in frying operations. At 405, information indicative of oil degradation can be obtained, such as using one or more of an optical or electrical sensor (e.g., an optical spectrometer or capacitive sensor). Such information may include data indicative of one or more of FFA, TPM, a color metric, polymer content, or other data indicative of edible oil degradation in a frying apparatus. At 410, model parameters can be obtained such as for use in applying an analytical model indicative of oil quality, as mentioned in relation to other examples herein. Such inputs may include attributes provided as inputs, or model parameters such as weighting factors, coefficients, calibration information, or a selection of an analytical model to be used for a specific use case). Use cases may be specified in terms of operating profiles as mentioned in relation to other examples herein. At 415, the model may be used to provide a quality index. The quality index may be compared against a threshold. In an illustrative example, if the quality index increases in magnitude in correspondence with oil degradation, then the technique 400 may involve triggering an action at 420 if the quality index exceeds the threshold. In another example, the technique 400 may involve triggering an action if the quality index deviates from a specified range of values. In another example, the technique 400 may involve binning the determined quality index value according to two or more specified ranges of values defining oil quality "bucket" categories or similar classifications. In yet another example, the technique 400 may involve triggering an action at 420 if the model indicates that the quality index will exceed a threshold or deviate from a specified range in the future, such as based on attribute information corresponding to anticipated future operations. At 420, the triggering an action may include one or more of triggering an oil top-off, removal of at least a portion of the oil in a frying apparatus, a filtration operation, or replacement of the oil. Such triggering can include generating a notification to a user via a display or providing some other indicium. In an example, operation of a fryer can be locked-out automatically if the action is not indicated as performed by the user (e.g., in an example a user must acknowledge the notification before operations may continue, or the user must perform the requested action and a sensor such as a switch (e.g., valve position sensor) or optical sensor can detect performance of the requested operation).

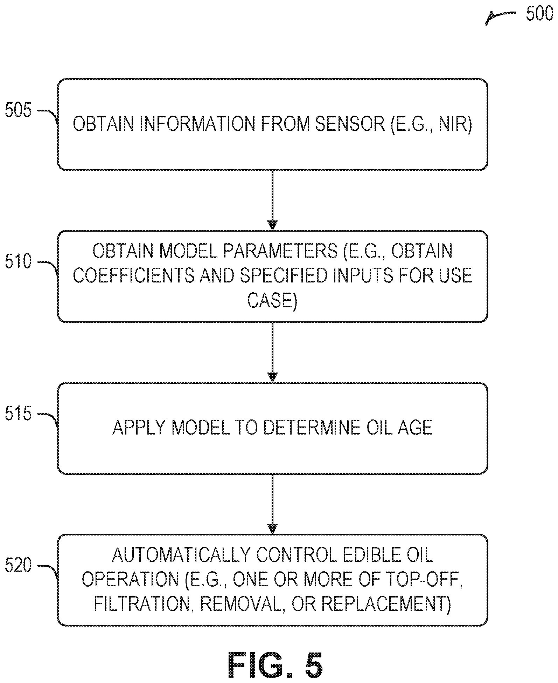

FIG. 5 illustrates generally a technique 500, such as a method, including use of an edible oil quality index to automatically control operations relating to managing edible oil used in frying operations. As in the example of FIG. 4, the technique 500 of FIG. 5 may include obtaining information at 505 from a sensor such as an optical spectrometer or a capacitive sensor. Again, as in the example of FIG. 4, the technique 500 of FIG. 5 may include obtaining model parameters such as attributes or other information specific to a use case and at 515 an analytical model indicative of an oil quality index can be applied such as to numerically determine an oil quality index, a range of such values, or a trend of such values. As in the example of FIG. 4, the technique 500 of FIG. 5 may compare the quality index value to a threshold or against a specified range, and at 520, the technique 500 may include automatically controlling one or more oil management operations in a frying apparatus. Such automatic control may include one or more of initiating or suppressing a top-off operation, a removal of at least a portion of the oil from fryer, a filtration operation, or even a replacement operation. In the example of FIG. 5, such automatic control may still be performed in the context of a "batch" frying operation, where at least some oil management operations may still involve operator interaction. Automation can include operation of valves such as to add or discharge oil, or triggering of other automation such as robotic or machine devices (e.g., to remove tubs of used oil or to perform other activities).

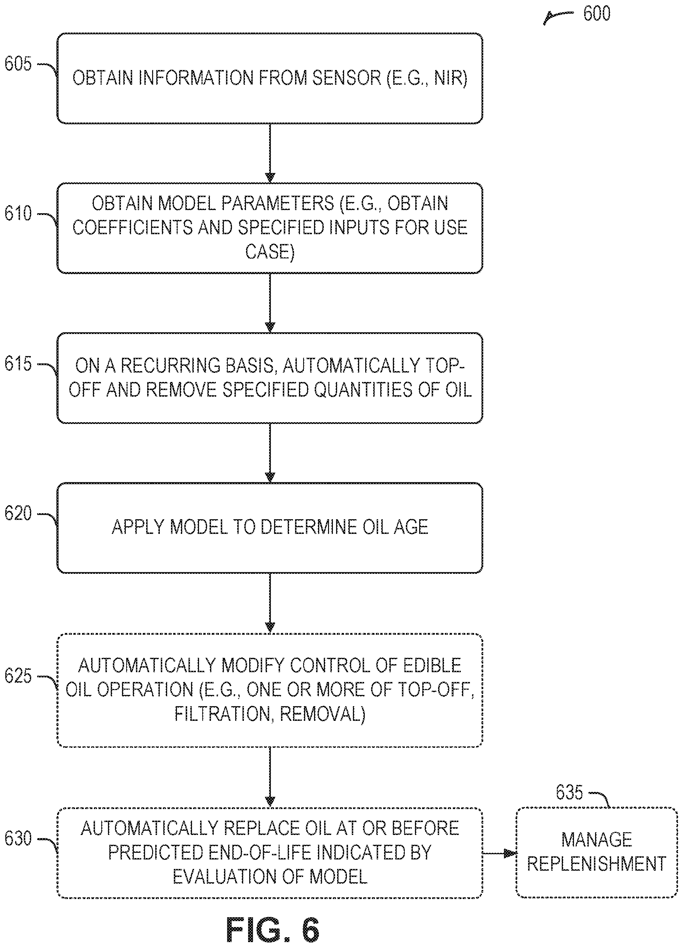

FIG. 6 illustrates generally a technique 600, such as a method, including use of an edible oil quality index to automatically control operations relating to managing edible oil used in frying operations, including automatically modifying control of edible oil operations. As in the examples of FIG. 4 and FIG. 5, at 605, information may be obtained from a sensor such as an optical spectrometer or a capacitive sensor. The information may include data indicative of oil degradation. At 610, model parameters such as coefficients, input or result weighting, or attribute information may be obtained to select or modify an analytical model indicative of edible oil quality, as in the examples of FIG. 4 and FIG. 5. At 615, top-off or oil removal operations may be initiated or suppressed, such as performed on a recurring basis under automatic control.

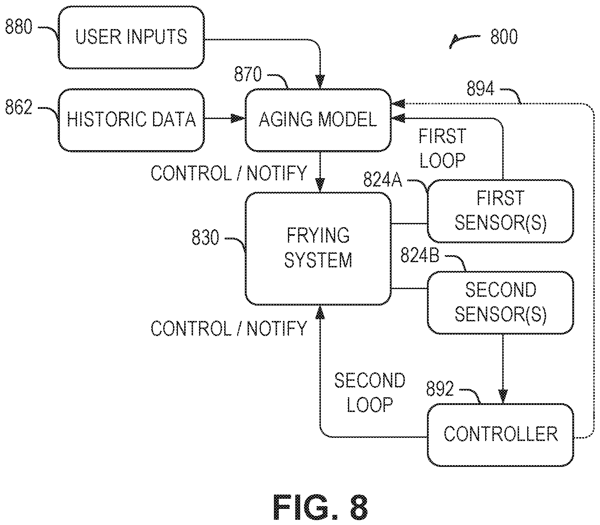

At 620, the analytical model may be applied to determine an oil quality index indicative of oil age. As in other examples, output from the model may be used by comparing the quality index against a threshold or for deviation from a specified range. At 625, in response to the application of the model at 620, one or more operational parameters relating edible oil management may be modified, such as adjusting one or more of the top-off, filtration, or removal operations. As an illustrative example, the technique 600 of FIG. 6 could include adding a specified top-off mass or volume of oil at specified intervals (such as hourly during a period where frying operations are occurring). If the oil vat level indicates that the oil level is trending higher, the top-off mass or volume could be adjusted to a lower value, or vice versa. Similarly, if the data obtained from the sensor indicative of oil degradation, such as TPM values, are trending higher, an oil replacement rate (e.g., a top-off frequency or volume, such as in combination with partial oil removal) may be increased, or vice versa. As another illustrative example, if an aldehyde level is less than a desired threshold or outside a desired range, an oil replacement rate may be decreased. Such examples are illustrative and many other variations may be used within the context of the technique 600. For example, a multi-loop scheme may be used as shown illustratively in FIG. 8.

Returning to FIG. 6, at 630, optionally, oil may be automatically replaced at or before a predicted end-of-life as indicated by the application (e.g., evaluation of numerical results) of the model at 620. At 635, replenishment may be managed, such as involved in generating a request for fresh oil to be delivered or dispatched, or for waste oil to be recovered for transport off-site to a re-processing or disposal facility. At 635, replenishment management need not be restricted to management of fresh oil supply or waste oil disposal. For example, attribute information provided as an input 610 may also include information indicative of a type or volume of food to be used or being used in frying operations. Replenishment management may include automatically triggering replenishment of food inventory based on attributes related to frying operations.

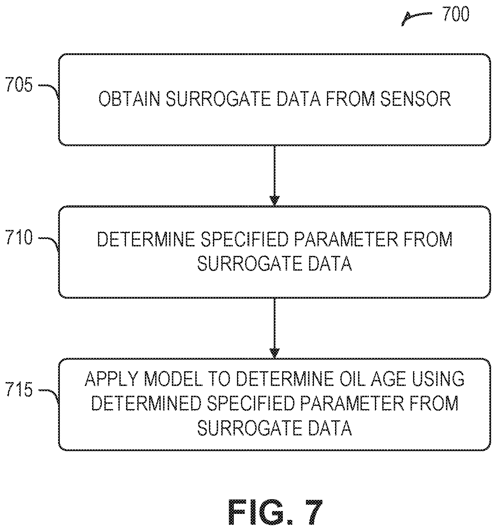

FIG. 7 illustrates generally a technique 700, such as a method, including use of surrogate data from a sensor to determine a parameter (e.g., an attribute) for use in determining an edible oil quality index. At 705, surrogate data can be obtained from a sensor. For example, if a basket drop event is not directly detectable in a frying apparatus (e.g., there is no switch or other mechanical or optical sensor indicating that a basket drop has occurred), then surrogate data can be obtained from another sensor. At 710, a specified parameter may be determined from the surrogate data. For example, temperature data may be obtained at 705, and a temperature profile versus time may be analyzed. If the temperature drops by more than a specified threshold, such as within a specified duration of time, then the temperature profile may be classified as indicative of a basket drop event, and a basket drop count may be incremented. Similarly, in another example, if an optical sensor or a capacitor sensor is used to obtain information indicative of oil degradation, such as a color metric, then at 705 the optical data can be obtained, and at 710 a specified parameter can be determined such as detecting that a filtration event has occurred. In another example, a type or temperature of food being fried can be determined at 710, such as by analyzing a time constant or other trend in a temperature profile obtained at 705. In yet another example, at 705, if the temperature is stable over a specified duration or below a specified threshold over a specified duration, then at 710 an attribute can be established indicating that the fryer is not being actively used and is in a holding mode, or vice versa.

FIG. 8 illustrates generally an example comprising a multi-loop control scheme 800 such as for use in managing edible oil used in frying operations. In FIG. 8, the frying system 830 may include a frying apparatus 130A as shown illustratively in FIG. 1 or variations thereof. In FIG. 8, as in other examples described herein, an analytical aging model can be applied to provide an oil quality index. The aging model can be selected, modified, or can receive as inputs for attributes, such as provided via user input 880. The aging model can be selected, modified, or can receive as inputs historical information 862 as mentioned in relation to other examples described herein. The frying system may also be monitored by one or more sensors such as a first sensor 824A (or sensors) defining a first control loop. The aging model 870 may be applied (e.g., to provide a numerical result), and such results may be used to provide one or more of notifications or control of operational parameters of the frying system 830. For example, the first sensor 824A may include one or more of an optical or electrical sensor to provide data indicative of oil degradation.

The frying system may also be managed using one or more other control loops, such as second control loop defined by a controller 892 and one or more other sensors such as second sensor 824B. The second loop can also provide one or more notifications or control of operational parameters. As an illustrative example, the second loop may be described as an oil level control loop, where the control information comprises control of an oil level in a vat of the frying system 830 including initiating top-offs or oil removal in a closed-loop manner based on oil level. The controller 892 can provide attribute information 894 to the aging model so that the aging model can factor in attribute data such as top-off occurrence, volume, duration, or frequency, as illustrative examples. In another example, the second loop may include a temperature regulation loop, and attribute data 894 provided to the aging mode can include data indicative that the fryer is being actively used versus holding, or that a temperature set point has been changed (such as indicative that the fryer is being used for par-frying versus other operations, as an example). In an example, the aging model 870 is used predictively to control the frying system 830 to maintain oil quality in view of approaching operational demand or particular approaching operational attributes, or to notify the operator that the oil in the frying system 830 will require attention.

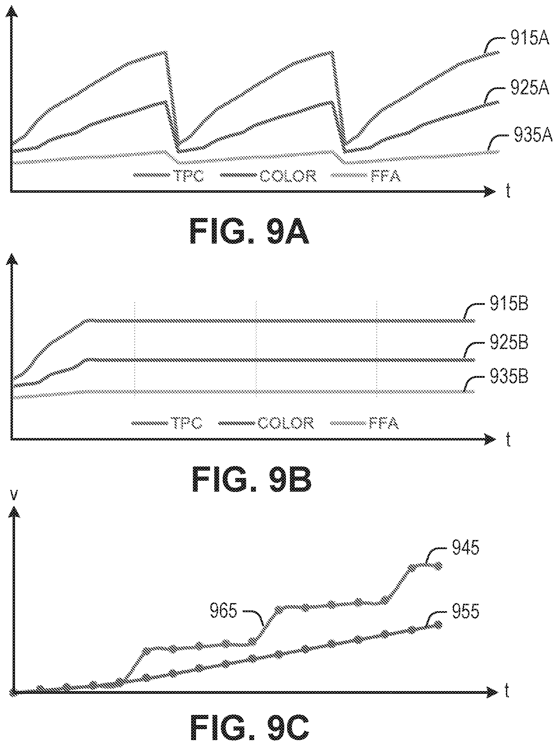

FIG. 9A illustrates generally a range of variation in various characteristics indicative of edible oil degradation, such as corresponding to an open-loop or go/no-go based monitoring scheme. As mentioned above, in the absence of the model-based examples described in this document, a wide variation is likely to occur in one or more characteristics of edible oil over the course of normal operations. For example, one or more of Total Polar Material (TPM 915A, a color metric 925A, or FFA 935A may vary widely across a range of values as oil is fresh, topped-off, then replaced again with fresh oil. By contrast, and without being bound by theory, the present inventors have recognized that through use of an analytical model taking into account attributes and sensed data, a reduced range of variation in various characteristics indicative of edible oil degradation can be achieved. For example, FIG. 9B illustrates generally targets for TPM 915B, a color metric 925B, and FFA 935B that may be achieved using an edible oil quality index to control operations relating to managing edible oil used in frying operations, such as by controlling top-off and oil removal in a semi-batch manner, for example.

FIG. 9C illustrates generally a comparison between a running cumulative total of oil volume 945 consumed using an open-loop or go/no-go monitoring scheme, as compared to a volume 955 corresponding to using an edible oil quality index to control operations relating to managing edible oil used in frying operations. At 955, because oil top-off and removal may be accomplished on an ongoing basis in a closed-loop manner, the cumulative volume of oil is lower. In the illustration of FIG. 9C, abrupt changes in the volume 945 are associated with oil replacement, which is believed to be required more frequently in the absence of the model-based monitoring or prediction techniques described herein.

An Illustrative Example Comprising a First Frying Oil Study

The examples of FIG. 9B and FIG. 9C show general illustrations indicating expected trends according to use of analytical model and sensed data. Generally, such techniques can be implemented and experimentally evaluated, such as by conducting a study under controlled conditions. In an illustrative example comprising a first frying oil study, a high-oleic canola oil (e.g., "HOCAN" oil) was subjected to a 15-day duration frying study. Four replicates were studied, with replicates labeled as "Oil 1," "Oil 2," "Oil 3," and "Oil 4," respectively. The study was conducted using Frymaster® (Shreveport, Louisiana, U.S.A.) fryers providing an oil capacity of 50 pounds per vat. The conditions for frying were a temperature set point of 350 Fahrenheit, 13.5 hour total heating time per day, a daily food-to-oil ratio of 0.4, and par-fried potatoes being fried. As oil was consumed during frying operations, fresh oil was added either once or twice daily as noted in the examples below. A mass of frying oil that was added each day is shown in TABLE 1 for a first set of replicates.

TABLE 1. Mass in pounds of frying oil added each day

| Day | Oil 1 | Oil 2 | Oil 3 | Oil 4 |
|-----|-------|-------|-------|-------|
| 0 | 50.00 | 50.00 | 50.00 | 50.00 |
| 1 | 18.20 | 17.20 | 14.00 | 14.20 |
| 2 | 14.80 | 15.40 | 15.00 | 15.00 |
| 3 | 15.60 | 16.80 | 18.60 | 19.60 |
| 4 | 20.20 | 18.80 | 19.00 | 22.40 |
| 5 | 16.40 | 21.20 | 20.00 | 16.40 |
| 6 | 18.40 | 20.10 | 18.10 | 16.20 |
| 7 | 19.20 | 19.00 | 18.40 | 18.80 |
| 8 | 1940 | 19.30 | 18.00 | 20.60 |
| 9 | 20.80 | 20.30 | 20.80 | |
| 10 | 18.20 | 17.00 | 16.60 | |
| 11 | 16.20 | 20.20 ! | 20.80 | |
| 12 | 20.60 | 20.50 | 20.80 | |
| 13 | 19.00 | 18.20 | 19.00 | |
| 14 | 16.30 | 17.30 | 18.30 | |

An oil quality index can be determined such as corresponding to an equivalent "age" of the oil in terms of hours of frying. Such an equivalent age can be determined using a mass-balance technique as discussed elsewhere in this document. For example, TABLE 2 illustrates an equivalent oil age in hours assuming that oil is added once per day. As shown in TABLE 2, such an equivalent oil age stabilizes after a short run-up period. Similarly, TABLE 3 illustrates an equivalent oil age in house assuming that oil is added twice per day (with half the allotted oil being added mid-day and half at the end of the day). In a manner similar to TABLE 2, the values in TABLE 3 stabilize, but at a slightly higher equivalent oil age value.

TABLE 2. Equivalent Oil Age in Hours when Oil Added Once Daily

| Hours | Day | Oil 1 | Oil 2 | Oil 3 | Oil 4 |
|---|---|---|---|---|---|
| 13.5 | 1 | 8.59 | 8.86 | 9.72 | 9.67 |
| 27 | 2 | 15.55 | 15.47 | 16.25 | 16.22 |
| 40.5 | 3 | 19.99 | 19.24 | 18.69 | 18.07 |
| 54 | 4 | 19.96 | 20.43 | 19.96 | 17.43 |
| 67.5 | 5 | 22.48 | 19.54 | 20.07 | 20.78 |
| 81 | 6 | 22.74 | 19.76 | 21.42 | 23.17 |
| 94.5 | 7 | 22.32 | 20.62 | 22.07 | 22.88 |
| 108 | 8 | 21.92 | 20.95 | 22.76 | 21.39 |
| 121.5 | 9 | 20.69 | 20.46 | 21.18 | |
| 135 | 10 | 21.74 | 22.42 | 23.17 | |
| 148.5 | 11 | 23.82 | 21.41 | 21.41 | |
| 162 | 12 | 21.95 | 20.59 | 20.39 | |
| 175.5 | 13 | 21.98 | 21.68 | 21.01 | |
| 189 | 14 | 23.91 | 23.01 | 21.88 | |

TABLE 3. Equivalent Oil Age in Hours when Oil Added Twice Daily

| Hours | Day | Oil 1 | Oil 2 | Oil 3 | Oil 4 |
|---|---|---|---|---|---|
| 13.5 | | 10.0 | 10.2 | 10.8 | 10.8 |
| 27 | 1 2 | 17.9 | 17.9 | 18.4 | 18.4 |
| 40.5 | 3 | 23.3 | 22.7 | 22.2 | 21.7 |
| 54 | 4 | 24.5 | 24.9 | 24.4 | 22.4 |
| 67.5 | 5 | 27.5 | 25.0 | 25.4 | 26.0 |
| 81 | 6 | 28.3 | 25.6 | 27.1 | 28.6 |
| 94.5 | 7 | 28.3 | 26.7 | 28.0 | 28.8 |
| 108 | 8 | 28.2 | 27.2 | 28.9 | 27.8 |
| 121.5 | 9 | 27.3 | 27.0 | 27.7 | |
| 135 | 10 | 28.3 | 28.8 | 29.6 | |
| 148.5 | 11 | 30.3 | 28.1 | 28.2 | |
| 162 | 12 | 28.7 | 27.4 | 27.2 | |
| 175.5 | 13 | 28.7 | 28.3 | 27.8 | |
| 189 | 14 | 30.5 | 29.6 | 28.6 | |

The results of this illustrative example comprising a first frying oil study illustrate that an oil quality index corresponding to an equivalent oil age (e.g., a weighted average based on mass balance or another technique) can be controlled, such as to achieve an equilibrium or stable age over time by controlling the addition of fresh oil. As discussed in relation to other examples herein, such as below, such an aging index can be related to sensed or measured parameters such as TPM, FFA, a color metric, or polymer content, as illustrative examples.

An Illustrative Example Comprising a Second Frying Oil Study

In an illustrative example comprising a second frying oil study, three different types of edible oil were independently subjected to a multi-week duration frying study. The three oil types included a soybean-based frying oil ("Soy"), a high-oleic canola oil ("HOCAN"), and palm olein. The study was conducted using new low-oil-volume (LOV) Frymaster®

(Shreveport, Louisiana, U.S.A.) fryers providing an oil capacity of 30 pounds per vat. The conditions for frying were a temperature set point of 350 Fahrenheit, with a 3-minute frying duration to fry Par-fried French fries. Filtration was performed using passive filtration at the end of each phase. Various measurements were made each day, to collect data indicative of TPM, FFA, and Color (Gardner Red).

The structure of this second frying oil study included a first phase prior to a achieving a measured TPM of greater-than-or-equal to 15% (e.g., corresponding to a target TPM value or a targeted TPM value range). Selection of a target TPM value or other attribute value can include targeting a range of values (e.g., 15%-17% or 15-20%, or another range), such as to provide desired taste or texture characteristics pertaining to enhancing or optimizing perceived food quality.

During this first phase, one 27-pound case of fries was fried per day per vat for each oil type and the oil was heated eight hours per day. This corresponds to a nominal daily food-to-oil ratio (FOR) of 0.9. Once a TPM value of 15% was achieved, a second phase was commenced spanning ten days. The second phase included continuing to fry one 27-pound case of fries per day per vat for each oil type, including removing oil (e.g., draining a controlled quantity of oil), then adding fresh oil to provide a measured TPM value of around 15% (e.g., by mass concentration). A determination of a quantity of oil to be drained can include use of an analytical expression as discussed below.

Upon completion of the ten-day duration, a third phase was commenced spanning another ten days. The third phase increased the nominal daily FOR from 0.9 to 1.8, corresponding to frying of two 27-pound cases of fries per day per vat. Again, oil was removed and fresh oil was added to control the TPM value to maintain a stabilized TPM value. Upon completion of the ten-day duration of the third phase, a fourth phase was commenced where the nominal daily FOR was increased from 1.8 to 3.6, corresponding to four 27-pound cases of fries being fried per day per vat for each oil type. During the fourth phase, it was unnecessary to drain oil because more oil was consumed (absorbed) by the increased volume of food being fried. Finally, the study was completed by returning to the one-case-per day rate used in the first and second phases. TABLE 4, below, illustrates various characteristics of the fresh oil used for each vat.

TABLE 4. Characteristics of the Fresh Oils used for the Second Study

|  | SOY | HOCAN | Palm Olein |
|---|---|---|---|
| Color [AOCS Red (5.25-inch)] | 1.2 | 1.4 | 3.3 |
| Free Fatty Acids as oleic (%) | 0.04 | 0.03 | 0.04 |
| 1 Peroxide Value [milliequivalents per kilogram; meq/kg] | 0.59 | 0.59 | 0.90 |
| Oil Stability Index (OSI) @ 110C [hours] | 8.04 | 15.37 | 22.15 |

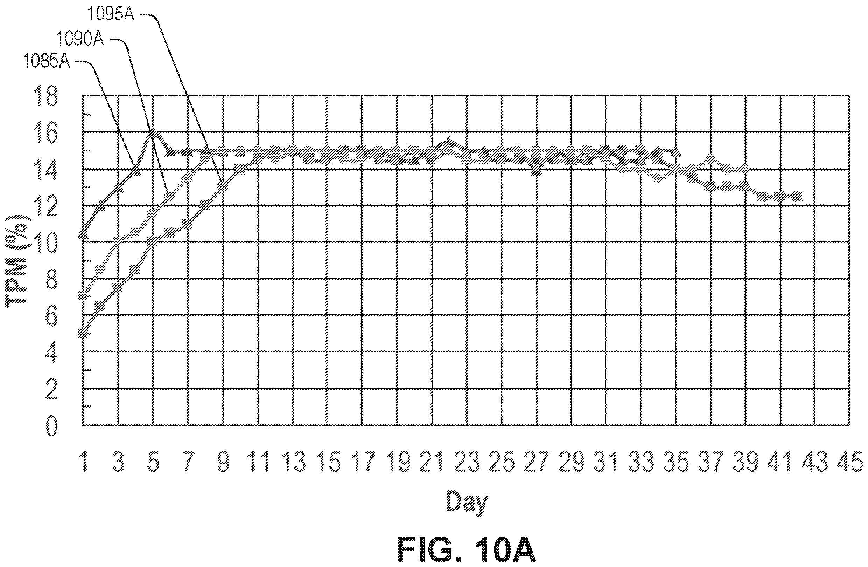

FIG. 10A illustrates generally experimentally-obtained measurements of Total Polar Material (TPM) percentages corresponding to three different frying oils over the course of the second frying oil study, according to an illustrative example. The plots shown in FIG. 10A correspond to the four phases of the second study as described above. The plot 1095A corresponds to HOCAN oil, the plot 1085A corresponds to Soy oil, and the plot 1090A corresponds to Palm Olein. In each plot, the measured TPM was allowed to climb to 15% (e.g., by mass concentration). Oil was drained and fresh oil was added to stabilize the TPM value. The plots 1085A, 1090A, and 1095A show that it is possible to achieve a stabilized TPM value across different oil types by adding fresh oil, and if needed, draining a specified quantity of used oil.

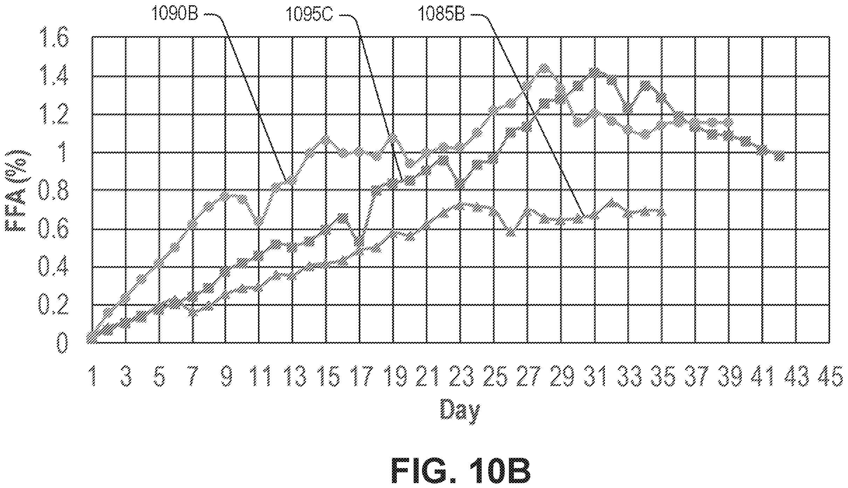

FIG. 10B illustrates generally experimentally-obtained measurements of Free Fatty Acid (FFA) percentages corresponding to the three different frying oils over the course of the second frying oil study, according to an illustrative example. In FIG. 10B, the day numbers correspond to the day numbering shown in FIG. 10A. The plot 1085B corresponds to Soy oil, the plot 1095B corresponds to HOCAN oil, and the plot 1090B corresponds to Palm Olein. Generally, in each of the plots 1085B, 1090B, and 10959B, FFA values increase and then stabilize, and in some examples, even decline (e.g., as more fresh oil was added due to the higher daily FOR versus other phases of the study).

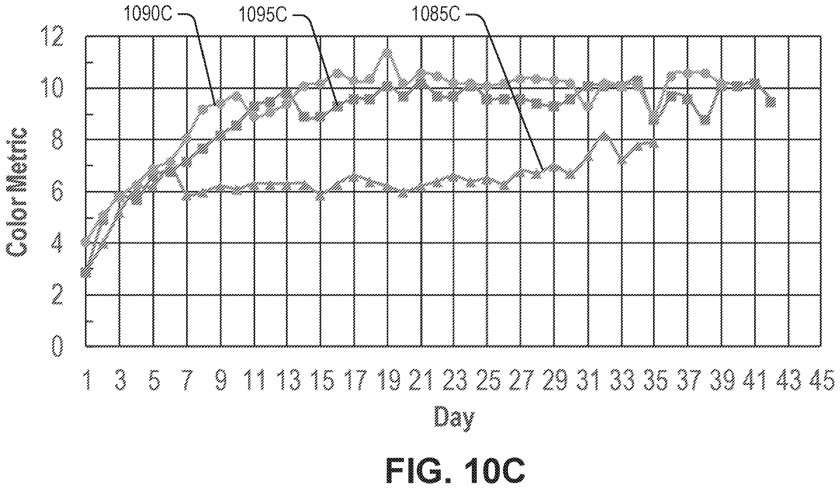

FIG. 10C illustrates generally experimentally-obtained measurements of a color metric corresponding to the three different frying oils over the course of the second frying oil study, according to an illustrative example. In FIG. 10C, the day numbers correspond to the day numbering shown in FIG. 10A and FIG. 10B. The plot 1085C corresponds to Soy oil, the plot 1095C corresponds to HOCAN oil, and the plot 1090C corresponds to Palm Olein. As in the examples of FIG. 10A and FIG. 10B, the values of the color metric generally increase and then stabilize, though the Soy oil plot 1085C shows an additional increase during the higher-volume phases of the study.

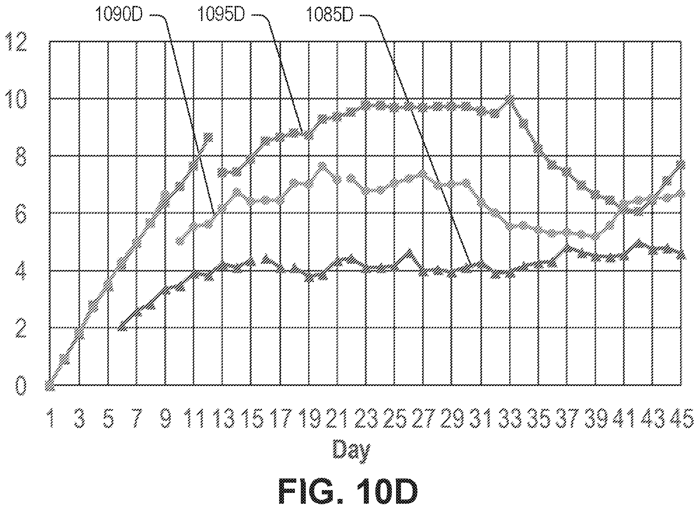

Generally, as mentioned in relation to other examples in this document, an oil quality index can be determined for the oil located in a vat (elsewhere), such as corresponding to an equivalent aging value of the oil. Such a determination can take into account oil that has been drained and oil that has been added. As an example, FIG. 10D illustrates generally an oil quality index represented as an equivalent age in days corresponding to the three different frying oils over the course of the study, according to an illustrative example.

The age values shown in FIG. 10D were determined by weighting the age of the oil in the vat in proportion to a mass of oil removed and a mass of fresh oil added to the vat. The age values can be related or weighted further using data obtained from a sensor (e.g., a TPM value, a color metric, a polymer content, or an FFA value, as illustrative examples). A summary of the overall FOR for the second study, along with FORs corresponding to each frying volume (one, two, and four cases per day) is shown below in TABLE 5. In another example, a TPM or other value can be estimated using the aging value, or vice versa.

As an example, a determination of a volume of oil to be removed (e.g., drained) or added can include using a measured or estimated volume of oil at the end of a frying duration (e.g., at the end of a day of frying or mid-day, as illustrative examples). Such a volume can be represented as "$V_1$," a maximum or target oil volume in the fryer (e.g., a vat capacity) can be represented as "$V_2$," a volume of used oil to be drained before top-off can be represented as "D," and a volume of fresh oil to be added can be represented as "T." A target value of a parameter such as TPM can be represented as "$P_2$," and a measured value of a parameter such as TPM can be represented as "$P_1$." According to an example, the following expression can be used to determine "D":

$$D = V_1 - V_2 \cdot (P_2/P_1).$$

If D≥0, then, the following expression can be used to determine "T":

$$T = V_2 - V_1 + D.$$

If D<0 (e.g., indicating that no drain operation is needed), then the following expression can be used to determine "T":

$$T = V_2 - V_1; \text{ which then provides } P_2 = P_1 \cdot (V_1/V_2).$$

In the latter expression, above, the $P_2$ value can represent an estimated value (e.g., of TPM) that would result after top-off. In the examples above, $V_1$ can be measured or calculated such as a function of one or more of a mass of food, a fat content before frying (e.g., expressed as a percentage, such as a percentage by mass), or a fat content after frying (also expressed as a percentage, such as a percentage by mass):

$$V_1 = f(M_{food}, FC_{before}, FC_{after})$$

In yet another example, a TPM value or other value can be predicted using an anticipated aging value. In yet another example, a TPM value or other value can be constrained such as by predicting a volume of oil to be one or more of added or removed, based on an aging value or other oil quality index.

TABLE 5. Summary of Oil Consumed, Food Fried, and FOR Values for Second Study

| | Total oil consumed (lb) | Food fried (lb) | Total FOR (entire study) | Actual FOR at nom. daily: FOR of 0.9 | Actual FOR at nom. daily FOR of 1.8 | Actual FOR at nom. daily FOR of 3.6 |
|---|---|---|---|---|---|---|
| **Palm Olein** | 222.22 | 2133.00 | 9.60 | 5.80 | 10.73 | 18.12 |
| **Soy** | 334.15 | 2025.00 | 6.06 | 3.18 | 5.36 | 14.36 |
| **HOCAN** | 189.60 | 2214.00 | 11.68 | 7.83 | 15.85 | 20.24 |

In a monitoring and control scheme as shown and described herein, which can be referred to as "continuous" frying, frying oil is discharged as needed and fresh oil is used to top off the fryer to maintain or improve oil quality. In this manner, the oil can be maintained at an intermediate age range, and more expensive oil may still be economical because oil consumption is lowered relative to less expensive oil types. Use of the phrase "continuous" does not require that frying itself occur continuously but refers to the use of a combination of used oil and top-off with fresh oil, and optional discharge of used oil, without requiring entirely changing out the oil in the vat as frequently as compared to other approaches.

Generally, Soy-derived oil is the least expensive oil per unit of the three oil types evaluated in the second study. However, the Soy oil was consumed at a far greater rate than either Palm Olein or HOCAN. Accordingly, even though Soy oil may be cheaper per unit, other oil types (e.g., Palm Olein or HOCAN) may provide equivalent or higher consistency in food quality and exhibited a much higher food-to-oil ratio when used within the context of a monitoring and control scheme as shown and described herein. Accordingly, an unexpected result of the present monitoring and control scheme is that more expensive oils may provide efficiencies in terms of oil consumption versus their cheaper counterparts.

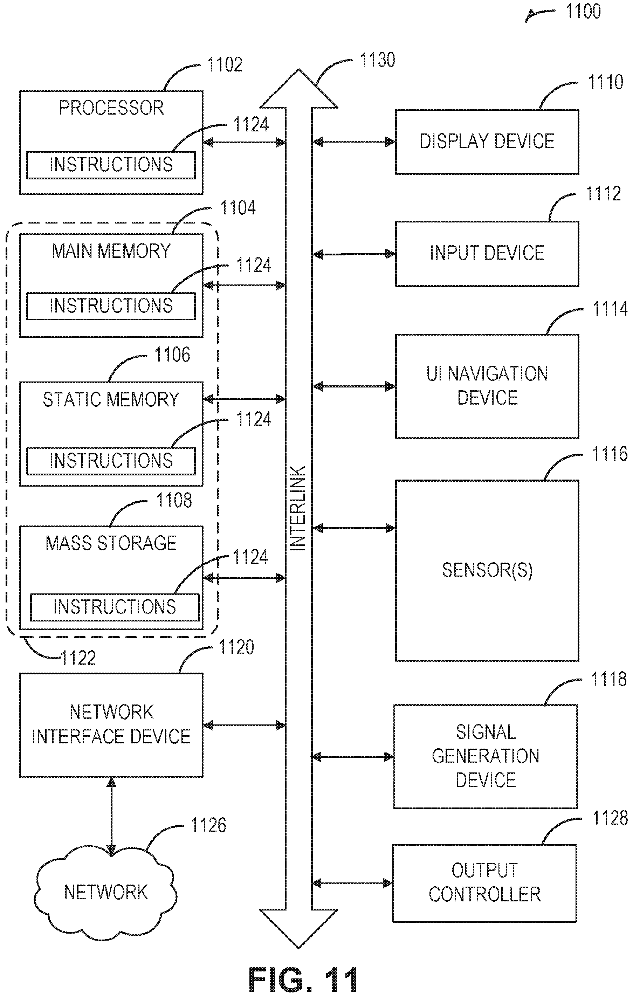

FIG. 11 illustrates a block diagram of an example comprising a machine 1100 upon which any one or more of the techniques (e.g., methodologies) discussed in this document may be performed. The machine 1100 may be included as a portion of elements shown in the system 100 of FIG. 1. In various examples, the machine 1100 may operate as a standalone device or may be connected (e.g., networked) to other machines. In a networked deployment, the machine 1100 may operate in the capacity of a server machine, a client machine, or both in server-client network environments. In an example, the machine 1100 may act as a peer machine in peer-to-peer (P2P) (or other distributed) network environment. The machine 1100 may be a personal computer (PC), a tablet device, a personal digital assistant (PDA), a mobile telephone, a web appliance, a network router, switch or bridge, a spectrometer or optical sensor such as including a microprocessor or microcontroller, or any machine capable of executing instructions (sequential or otherwise) that specify actions to be taken by that machine. Further, while only a single machine is illustrated, the term "machine" shall also be taken to include any collection of machines that individually or jointly execute a set (or multiple sets) of instructions to perform any one or more of the methodologies discussed herein, such as cloud computing, software as a service (SaaS), other computer cluster configurations.

Examples, as described herein, may include, or may operate by, logic or a number of components, or mechanisms. "Circuitry" refers generally a collection of circuits implemented in tangible entities that include hardware (e.g., simple circuits, gates, logic elements, etc.). Circuitry membership may be flexible over time and underlying hardware variability. Circuitries include members that may, alone or in combination, perform specified operations when operating. In an example, hardware of the circuitry may be immutably designed to carry out a specific operation (e.g., hardwired). In an example, the hardware comprising the circuitry may include variably connected physical components (e.g., execution units, transistors, simple circuits, etc.) including a computer readable medium physically modified (e.g., magnetically, electrically, such as via a change in physical state or transformation of another physical characteristic, etc.) to encode instructions of the specific operation.

In connecting the physical components, the underlying electrical properties of a hardware constituent may be changed, for example, from an insulating characteristic to a conductive characteristic or vice versa. The instructions enable embedded hardware (e.g., the execution units or a loading mechanism) to create members of the circuitry in hardware via the variable connections to carry out portions of the specific operation when in operation. Accordingly, the computer readable medium is communicatively coupled to the other components of the circuitry when the device is operating. In an example, any of the physical components may be used in more than one member of more than one circuitry. For example, under operation, execution units may be used in a first circuit of a first circuitry at one point in time and reused by a second circuit in the first circuitry, or by a third circuit in a second circuitry at a different time.

Machine (e.g., computer system) 1100 may include a hardware processor 1102 (e.g., a central processing unit (CPU), a graphics processing unit (GPU), a hardware processor core, or any combination thereof), a main memory 1104 and a static memory 1106, some or all of which may communicate with each other via an interlink (e.g., bus) 1108. The machine 1100 may further include a display unit 1110, an alphanumeric input device 1112 (e.g., a keyboard), and a user interface (UI) navigation device 1114 (e.g., a mouse). In an example, the display unit 1110, input device 1112 and UI navigation device 1114 may be a touch screen display. The machine 1100 may additionally include a storage device (e.g., drive unit) 1116, a signal generation device 1118 (e.g., a speaker), a network interface device 1120, and one or more sensors 1121, such as a global positioning system (GPS) sensor, compass, accelerometer, or other sensor. The machine 1100 may include an output controller 1128, such as a serial (e.g., universal serial bus (USB), parallel, or other wired or wireless (e.g., infrared (IR), near field communication (NFC), etc.) connection to communicate or control one or more peripheral devices (e.g., a printer, card reader, etc.).

The storage device 1116 may include a machine readable medium 1122 on which is stored one or more sets of data structures or instructions 1124 (e.g., software) embodying or utilized by any one or more of the techniques or functions described herein. The instructions 1124 may also reside, completely or at least partially, within the main memory 1104, within static memory 1106, or within the hardware processor 1102 during execution thereof by the machine 1100. In an example, one or any combination of the hardware processor 1102, the main memory 1104, the static memory 1106, or the storage device 1116 may constitute machine readable media.

While the machine readable medium 1122 is illustrated as a single medium, the term "machine readable medium" may include a single medium or multiple media (e.g., a centralized or distributed database, and/or associated caches and servers) configured to store the one or more instructions 1124.

The term "machine readable medium" may include any medium that is capable of storing, encoding, or carrying instructions for execution by the machine 1100 and that cause the machine 1100 to perform any one or more of the techniques of the present disclosure, or that is capable of storing, encoding or carrying data structures used by or associated with such instructions. Non-limiting machine readable medium examples may include solid-state memories, and optical and magnetic media. Accordingly, machine-readable media are not transitory propagating signals. Specific examples of massed machine readable media may include: non-volatile memory, such as semiconductor memory devices (e.g., Electrically Programmable Read-Only Memory (EPROM), Electrically Erasable Programmable Read-Only Memory (EEPROM)) and flash memory devices; magnetic or other phase-change or state-change memory circuits; magnetic disks, such as internal hard disks and removable disks; magneto-optical disks; and CD-ROM and DVD-ROM disks.

The instructions 1124 may further be transmitted or received over a communications network 1126 using a transmission medium via the network interface device 1120 utilizing any one of a number of transfer protocols (e.g., frame relay, internet protocol (IP), transmission control protocol (TCP), user datagram protocol (UDP), hypertext transfer protocol (HTTP), etc.). Example communication networks may include a local area network (LAN), a wide area network (WAN), a packet data network (e.g., the Internet), mobile telephone networks (e.g., cellular networks such as conforming to one or more standards such as a 4G standard or Long Term Evolution (LTE)), Plain Old Telephone (POTS) networks, and wireless data networks (e.g., Institute of Electrical and Electronics Engineers (IEEE) 802.11 family of standards known as Wi-Fi®, IEEE 802.15.4 family of standards, peer-to-peer (P2P) networks, among others. In an example, the network interface device 1120 may include one or more physical jacks (e.g., Ethernet, coaxial, or phone jacks) or one or more antennas to connect to the communications network 1126. In an example, the network interface device 1120 may include a plurality of antennas to wirelessly communicate using at least one of single-input multiple-output (SIMO), multiple-input multiple-output (MIMO), or multiple-input single-output (MISO) techniques. The term "transmission medium" shall be taken to include any intangible medium that is capable of storing, encoding or carrying instructions for execution by the machine 1100, and includes digital or analog communications signals or other intangible medium to facilitate communication of such software.

Various Notes

In Example 1, a computer-implemented or other automated method for monitoring a characteristic of edible oil can include using an optical technique, to manage oil quality in frying operations, the method including obtaining a sensed characteristic of edible oil in a frying apparatus, using an optical sensor, receiving data indicative of an attribute other than obtained using the optical sensor, automatically determining an edible oil quality index using an analytical model, the analytical model defined as a function of the sensed characteristic and the attribute, and in response to determining the edible oil quality index, automatically triggering an action.

In Example 2, the method of Example 1 can optionally include triggering an action includes providing an indication to perform at least one of: a top-off of the edible oil in the frying apparatus; a filtration of edible oil in the frying apparatus; or removal of at least a portion of the edible oil from the frying apparatus.

In Example 3, the method of Example 2 can optionally include that the triggering the action includes automatically triggering at least one of: a top-off of the edible oil in the frying apparatus; a filtration of edible oil in the frying apparatus; or removal of at least a portion of the edible oil from the frying apparatus without requiring operator intervention.

In Example 4, the method of any of Examples 1 through 3 can optionally include that the triggering the action occurs in response to a comparison between the determined edible oil quality index and a specified threshold.

In Example 5, the method of any of Examples 1 through 4 can optionally include triggering the action to maintain the edible oil within a specified range of quality index values.

In Example 6, the method of any of Examples 1 through 5 can optionally include that the optical sensor comprises a near-infrared optical sensor arranged to perform at least one of: an optical transmission measurement through the edible oil, or an optical reflectance measurement from a surface of the oil.

In Example 7, the method of any of Examples 1 through 6 can optionally include that the optical sensor comprises a spectrometer.

In Example 8, the method of any of Examples 1 through 7 can optionally include that the attribute comprises data indicative of a temporal attribute, including at least one of: a time of day, a time of week, a time of month, a time of year, a duration since last oil replacement, a duration since last oil filtration, or a duration since a last fryer cleaning.

In Example 9, the method of Example 8 can optionally include that the attribute comprises a fryer, food, store, or operator attribute specified in terms of the temporal attribute.

In Example 10, the method of any of Examples 1 through 9 can optionally include that triggering the action includes at least one of: automatically controlling a valve to provide fresh edible oil to the frying apparatus, or automatically controlling a valve to remove at least a portion of used edible oil from the frying apparatus.

In Example 11, the method of any of Examples 1 through 10 can optionally include monitoring a fresh oil source, and in response to the monitoring, triggering replenishment of the fresh oil source when the monitoring indicates that a level of the fresh oil source is below a specified threshold.

In Example 12, the method of Example 11 can optionally include that the triggering the replenishment includes transmitting a replenishment request to a recipient in the edible oil supply chain.

In Example 13, the method of any of Examples 1 through 12 can optionally include determining a trend in the edible oil quality index by applying the analytical model and predicting at least one or an oil consumption metric or a waste oil metric, using the determined trend.

In Example 14, the method of any of Examples 1 through 13 can optionally include that the attribute comprises a sensed attribute including at least one of: a temperature, a count of basket drops, or a count of filtration operations.

In Example 15, the method of Example 14 can optionally include that the sensed attribute comprises a surrogate for another attribute.

In Example 16, the method of Example 14 can optionally include the sensed attribute comprises a temperature profile versus time and the method comprises determining an occurrence of a basket drop using the sensed temperature profile.

In Example 17, the method of any of Examples 1 through 16 can optionally include that the quality index is correlated with at least one of: a free fatty acid (FFA) value, a Gardner color unit, a total polar material (TPM) level, or a polymer content.

In Example 18, a computer-implemented or other automated method for monitoring a characteristic of edible oil to manage oil quality in frying operations can include obtaining a sensed characteristic of edible oil in a frying apparatus using a sensor, the sensed characteristic indicative of edible oil degradation, receiving data indicative of an attribute other than obtained using the sensor, the attribute defining a future operational condition for frying operations, automatically determining a predicted edible oil quality index using an analytical model, the analytical model defined as a function of the sensed characteristic and the attribute, and in response to determining the predicted edible oil quality index, triggering an action.

In Example 19, the method of Example 18 can optionally include that the sensor comprises an optical sensor.

In Example 20, the method of any of Examples 18 through 19 can optionally include that the optical sensor comprises a near-infrared optical spectrometer.

In Example 21, the method of any of Examples 18 through 20 can optionally include that the sensor comprises an electrical sensor.

In Example 22, the method of any of Examples 18 through 21 can optionally include that the sensor comprises a capacitive sensor.

In Example 23, the method of any of Examples 18 through 22 can optionally include that the sensed characteristic includes at least one of: a free fatty acid (FFA) value, a Gardner color unit, a total polar material (TPM) level, or a polymer content.

In Example 24, the method of any of Examples 18 through 23 can optionally include that the attribute defining a future operational condition comprises data indicative of a temporal attribute, including at least one of: a time of day, a time of week, a time of month, a time of year, a duration since last oil replacement, a duration since last oil filtration, or a duration since a last fryer cleaning.

In Example 25, the method of Example 24 can optionally include that the attribute comprises a fryer, food, store, or operator attribute specified in terms of the temporal attribute.

In Example 26, the method of any of Examples 18 through 25 can optionally include that triggering the action includes at least one of: automatically controlling a valve to provide fresh edible oil to the frying apparatus, or automatically controlling a valve to remove at least a portion of used edible oil from the frying apparatus.

In Example 27, the method of any of Examples 18 through 26 can optionally include automatically initiating oil top-off operations or removal operations of edible oil from a frying vat in response to the determined predicted quality index.

In Example 28, the method of Example 27 can optionally include that a volume or frequency of edible oil top-off operations is adjusted in response to the predicted quality index.

In Example 29, the method of Example 27 can optionally include that a volume or frequency of edible oil removal operations is adjusted in response to the predicted quality index.

In Example 30, the method of Example 27 can optionally include that a volume or frequency of edible oil top-off operations is adjusted in response to a determined trend of the predicted quality index.

In Example 31, the method of Example 27 can optionally include that a volume or frequency of edible oil removal operations is adjusted in response to a determined trend of the predicted quality index.

In Example 32, the method of Example 27 can optionally include modifying at least one the edible oil top-off operations or removal operations of edible oil in response to a change in the attribute defining a future operational condition for frying operations.

In Example 33, an apparatus such as a system can include a frying oil reservoir containing edible oil, a sensor to obtain a sensed characteristic indicative of degradation of the edible oil, a controller configured to receive data indicative of an attribute other than obtained using the sensor, along with the sensed characteristic, the controller configured to determine a predicted edible oil quality index using an analytical model, the analytical model defined as a function of the sensed characteristic and the attribute, and in response to determining the predicted edible oil quality index, the controller configured to trigger an action including one or more of top-off of the edible oil, removal of at least a portion of the edible oil, filtration of the edible oil, or replacement of the edible oil.

In Example 34, the apparatus of Example 33 can optionally include a valve located between an oil source and the oil vat, where the controller is communicatively coupled to the valve, and where the controller is configured to control the valve to provide fresh oil to the oil vat in response to determining the predicted edible oil quality index.

In Example 35, the apparatus of any of Examples 33 through 34 can optionally include a valve located between a waste oil repository and the oil vat, where the controller is communicatively coupled to the valve, and where the controller is configured to control the valve to provide remove oil from the oil vat in response to determining the predicted edible oil quality index.

In Example 36, the apparatus of any of Examples 33 through 35 can optionally include that the sensor comprises an optical sensor.

In Example 37, the apparatus of any of Examples 33 through 36 can optionally include that the sensor comprises an infra-red spectrometer.

In Example 38, the apparatus of any of Examples 33 through 37 can optionally include that the sensor is hand-held and is wirelessly communicatively coupled to the controller.

In Example 39, a computer-implemented or other automated method for monitoring a characteristic to manage edible oil use in frying operations can include receiving data indicative of an attribute of edible oil used in frying apparatus, automatically applying an analytical model indicative of an oil quality index to provide a predicted oil consumption metric, the analytical model defined as a function of a sensed characteristic and the attribute, and in response to applying the analytical model, triggering an action based on a predicted oil consumption metric.

In Example 40, the method of Example 39 can optionally include that the sensed characteristic is indicative of a volume of oil in the frying apparatus or a trend of such volume, where the attribute comprises a temporal attribute.

In Example 41, the method of Example 40 can optionally include that the temporal attribute is used to select one or more of a particular analytical model instance or a parameter of the analytical model.

In Example 42, the method of any of Examples 39 through 41 can optionally include that the temporal attribute comprises at least one of: a time of day, a time of week, a time of month, a time of year, a duration since last oil replacement, a duration since last oil filtration, or a duration since a last fryer cleaning.

In Example 43, the method of any of Examples 39 through 42 can optionally include determining a trend in the edible oil quality index by applying the analytical model and predicting at least one of: an oil consumption metric or a waste oil metric, using the determined trend.

In Example 44, the method of any of Examples 39 through 43 can optionally include that the sensed characteristic includes at least one of: a temperature, an oil level, a count of basket drops, or a count of filtration operations.

In Example 45, the method of any of Examples 39 through 44 can optionally include that the sensed characteristic comprises a surrogate for another attribute.

In Example 46, the method of any of Examples 39 through 45 can optionally include that the triggering an action includes triggering at least one of: a top-off of the edible oil in the frying apparatus; a filtration of edible oil in the frying apparatus; or removal of at least a portion of the edible oil from the frying apparatus.

Each of the non-limiting aspects described herein may stand on its own, or may be combined in various permutations or combinations with one or more of the other aspects or other subject matter described in this document.

The above detailed description includes references to the accompanying drawings, which form a part of the detailed description. The drawings show, by way of illustration, specific embodiments in which the invention may be practiced. These embodiments are also referred to generally as "examples." Such examples may include elements in addition to those shown or described. However, the present inventors also contemplate examples in which only those elements shown or described are provided. Moreover, the present inventors also contemplate examples using any combination or permutation of those elements shown or described (or one or more aspects thereof), either with respect to a particular example (or one or more aspects thereof), or with respect to other examples (or one or more aspects thereof) shown or described herein.

Method examples described herein may be machine or computer-implemented at least in part. Some examples may include a computer-readable medium or machine-readable medium encoded with instructions operable to configure an electronic device to perform methods as described in the above examples. An implementation of such methods may include code, such as microcode, assembly language code, a higher-level language code, or the like. Such code may include computer readable instructions for performing various methods. The code may form portions of computer program products. Further, in an example, the code may be tangibly stored on one or more volatile, non-transitory, or non-volatile tangible computer-readable media, such as during execution or at other times. Examples of these tangible computer-readable media may include, but are not limited to, hard disks, removable magnetic disks, removable optical disks (e.g., compact disks and digital video disks), magnetic cassettes, memory cards or sticks, random access memories (RAMs), read only memories (ROMs), and the like.

The above description is intended to be illustrative, and not restrictive. For example, the above-described examples (or one or more aspects thereof) may be used in combination with each other. Other embodiments may be used, such as by one of ordinary skill in the art upon reviewing the above description. The scope of the invention should be determined with reference to the appended claims.

# EP 3 902 453 B1

## Claims

1. A method for monitoring a characteristic of edible oil using an optical technique, to manage oil quality in frying operations, the method comprising:

   obtaining a sensed characteristic of edible oil in a frying apparatus, using an optical sensor;
   receiving data indicative of an attribute other than obtained using the optical sensor;
   automatically determining an edible oil quality index using an analytical model, the analytical model defined as a function of the sensed characteristic and the attribute; and
   in response to determining the edible oil quality index, automatically triggering an action, wherein the method is **characterized in** comprising:

   determining a trend in the edible oil quality index by applying the analytical model; and
   predicting at least one of: an oil consumption metric or a waste oil metric, using the determined trend.

2. The method of claim 1, wherein the triggering an action includes providing an indication to perform at least one of: a top-off of the edible oil in the frying apparatus; a filtration of edible oil in the frying apparatus; or removal of at least a portion of the edible oil from the frying apparatus.

3. The method of claim 2, wherein triggering the action includes automatically triggering at least one of: a top-off of the edible oil in the frying apparatus; a filtration of edible oil in the frying apparatus; or removal of at least a portion of the edible oil from the frying apparatus without requiring operator intervention.

4. The method of claim 1, wherein the triggering the action occurs in response to a comparison between the determined edible oil quality index and a specified threshold.

5. The method of claim 1, wherein the method comprises triggering the action to maintain the edible oil within a specified range of quality index values.

6. The method of claim 1, wherein the optical sensor comprises a near-infrared optical sensor arranged to perform at least one of: an optical transmission measurement through the edible oil, or an optical reflectance measurement from a surface of the oil.

7. The method of claim 1, wherein the attribute comprises data indicative of a temporal attribute, including at least one of: a time of day, a time of week, a time of month, a time of year, a duration since last oil replacement, a duration since last oil filtration, or a duration since a last fryer cleaning.

8. The method of claim 1, wherein triggering the action includes at least one of:

   automatically controlling a valve to provide fresh edible oil to the frying apparatus, or
   automatically controlling a valve to remove at least a portion of used edible oil from the frying apparatus.

9. The method of claim 1, comprising monitoring a fresh oil source; and
   in response to the monitoring, triggering replenishment of the fresh oil source when the monitoring indicates that a level of the fresh oil source is below a specified threshold.

10. The method of claim 9, wherein triggering the replenishment includes transmitting a replenishment request to a recipient in the edible oil supply chain.

11. The method of claim 1, wherein the quality index is correlated with at least one of: a free fatty acid (FFA) value, a Gardner color unit, a total polar material (TPM) level, or a polymer content.

## Patentansprüche

1. Verfahren zum Überwachen einer Eigenschaft von Speiseöl unter Verwendung einer optischen Technik, um die Ölqualität bei Frittiervorgängen zu verwalten, das Verfahren umfassend:

Erhalten einer erfassten Eigenschaft von Speiseöl in einer Frittiervorrichtung unter Verwendung eines optischen Sensors;

Empfangen von anderen Daten, die für ein Attribut kennzeichnend sind, als die die unter Verwendung des optischen Sensors erhalten werden;

automatisches Bestimmen eines Speiseölqualitätsindex unter Verwendung eines analytischen Modells, wobei das analytische Modell in Abhängigkeit von der erfassten Eigenschaft und dem Attribut definiert ist; und

als Reaktion auf das Bestimmen des Speiseölqualitätsindexes, automatisches Auslösen einer Aktion, wobei das Verfahren

**dadurch gekennzeichnet ist, dass** es umfasst:

Bestimmen eines Trends in dem Speiseölqualitätsindex durch Anwenden des analytischen Modells; und

Vorhersagen von mindestens einem von: einer Ölverbrauchskennzahl oder einer Altölkennzahl unter Verwendung des bestimmten Trends.

2.  Verfahren nach Anspruch 1, wobei das Auslösen einer Aktion das Bereitstellen eines Kennzeichens zum Durchführen von mindestens einem einschließt von: einem Nachfüllen des Speiseöls in der Frittiervorrichtung; einer Filtration von Speiseöl in der Frittiervorrichtung; oder Entfernen mindestens eines Anteils des Speiseöls aus der Frittiervorrichtung.

3.  Verfahren nach Anspruch 2, wobei das Auslösen der Aktion das automatische Auslösen von mindestens einem einschließt von: einem Nachfüllen des Speiseöls in der Frittiervorrichtung; einer Filtration von Speiseöl in der Frittiervorrichtung; oder Entfernen von mindestens einem Anteil des Speiseöls aus der Frittiervorrichtung, ohne dass ein Eingreifen des Bedieners erforderlich ist.

4.  Verfahren nach Anspruch 1, wobei das Auslösen der Aktion als Reaktion auf einen Vergleich zwischen dem bestimmten Speiseölqualitätsindex und einem spezifischen Schwellenwert erfolgt.

5.  Verfahren nach Anspruch 1, wobei das Verfahren das Auslösen der Aktion umfasst, um das Speiseöl innerhalb eines spezifischen Bereichs von Qualitätsindexwerten zu halten.

6.  Verfahren nach Anspruch 1, wobei der optische Sensor einen optischen Nahinfrarotsensor umfasst, der so angeordnet ist, um mindestens eines durchzuführen von: einer optische Transmissionsmessung durch das Speiseöl oder einer optische Reflexionsmessung von einer Oberfläche des Öls.

7.  Verfahren nach Anspruch 1, wobei das Attribut Daten umfasst, die für ein zeitliches Attribut kennzeichnend sind, einschließlich mindestens eines von: einer Tageszeit, einer Wochenzeit, einer Monatszeit, einer Jahreszeit, einer Zeitspanne seit dem letzten Ölwechsel, einer Zeitspanne seit der letzten Ölfiltration oder einer Zeitspanne seit der letzten Fritteusenreinigung.

8.  Verfahren nach Anspruch 1, wobei das Auslösen der Aktion mindestens eines einschließt von: automatischem Steuern eines Ventils, um der Frittiervorrichtung frisches Speiseöl bereitzustellen, oder automatisches Steuern eines Ventils, um mindestens einen Anteil des verwendeten Speiseöls aus der Frittiervorrichtung zu entfernen.

9.  Verfahren nach Anspruch 1, umfassend das Überwachen einer Frischölquelle; und

als Reaktion auf das Überwachen, Auslösen einer Nachfüllung der Frischölquelle, wenn die Überwachung kennzeichnet, dass ein Pegel der Frischölquelle unter einem spezifischen Schwellenwert liegt.

10. Verfahren nach Anspruch 9, wobei das Auslösen der Nachfüllung das Übermitteln einer Nachfüllanforderung an einen Empfänger in der Lieferkette für Speiseöl einschließt.

11. Verfahren nach Anspruch 1, wobei der Qualitätsindex mit mindestens einem korreliert ist von: einem Wert für freie Fettsäuren (FFA), einer Gardner-Farbeinheit, einem Gesamtpegel an polarem Material (TPM) oder einem polymeren Inhalt.

**Revendications**

1.  Procédé de surveillance d'une caractéristique d'huile comestible à l'aide d'une technique optique, pour gérer la qualité de l'huile dans des opérations de friture, le procédé comprenant :

l'obtention d'une caractéristique détectée d'huile comestible dans un appareil de friture, à l'aide d'un capteur optique ;
la réception de données indiquant un attribut autre que celui obtenu à l'aide du capteur optique ;
la détermination automatique d'un indice de qualité d'huile comestible à l'aide d'un modèle analytique, le modèle analytique étant défini en fonction de la caractéristique détectée et de l'attribut ; et
en réponse à la détermination de l'indice de qualité d'huile comestible, le déclenchement automatique d'une action, dans lequel le procédé est **caractérisé en ce qu'**il comprend :

la détermination d'une tendance dans l'indice de qualité d'huile comestible en appliquant le modèle analytique ; et
la prédiction d'au moins l'une parmi : une métrique de consommation d'huile ou une métrique d'huile usée, à l'aide de la tendance déterminée.

2. Procédé selon la revendication 1, dans lequel le déclenchement d'une action comporte la fourniture d'une indication pour effectuer au moins l'un parmi : un appoint de l'huile comestible dans l'appareil de friture ; une filtration de l'huile comestible dans l'appareil de friture ; ou l'élimination d'au moins une partie de l'huile comestible de l'appareil de friture.

3. Procédé selon la revendication 2, dans lequel le déclenchement de l'action comporte le déclenchement automatique d'au moins l'un parmi : un appoint de l'huile comestible dans l'appareil de friture ; une filtration de l'huile comestible dans l'appareil de friture ; ou l'élimination d'au moins une partie de l'huile comestible de l'appareil de friture sans nécessiter d'intervention de l'opérateur.

4. Procédé selon la revendication 1, dans lequel le déclenchement de l'action se produit en réponse à une comparaison entre l'indice de qualité d'huile comestible déterminé et un seuil spécifié.

5. Procédé selon la revendication 1, dans lequel le procédé comprend le déclenchement de l'action pour maintenir l'huile comestible au sein d'une plage spécifiée de valeurs d'indice de qualité.

6. Procédé selon la revendication 1, dans lequel le capteur optique comprend un capteur optique proche infrarouge agencé pour effectuer au moins l'une parmi : une mesure de transmission optique à travers l'huile comestible, ou une mesure de réflectance optique à partir d'une surface de l'huile.

7. Procédé selon la revendication 1, dans lequel l'attribut comprend des données indiquant un attribut temporel, y compris au moins l'une parmi : une heure du jour, une heure de la semaine, une heure du mois, une heure de l'année, une durée depuis le dernier remplacement d'huile, une durée depuis la dernière filtration d'huile, ou une durée depuis le dernier nettoyage de friteuse.

8. Procédé selon la revendication 1, dans lequel le déclenchement de l'action comporte au moins l'une parmi : la commande automatique d'une vanne pour fournir de l'huile comestible fraîche à l'appareil de friture, ou la commande automatique d'une vanne pour éliminer au moins une partie de l'huile comestible usagée de l'appareil de friture.

9. Procédé selon la revendication 1, comprenant la surveillance d'une source d'huile fraîche ; et
en réponse à la surveillance, le déclenchement du réapprovisionnement de la source d'huile fraîche lorsque la surveillance indique qu'un niveau de la source d'huile fraîche est inférieur à un seuil spécifié.

10. Procédé selon la revendication 9, dans lequel le déclenchement du réapprovisionnement comporte la transmission d'une demande de réapprovisionnement à un destinataire de la chaîne d'approvisionnement en huile comestible.

11. Procédé selon la revendication 1, dans lequel l'indice de qualité est corrélé avec au moins l'un parmi : une valeur d'acide gras libre (AGL), une unité de couleur Gardner, un niveau de matières polaires totales (TPM) ou une teneur en polymère.

FIG. 1

250 — OIL ATTRIBUTE INPUTS

252 — FRYER ATTRIBUTE INPUTS

254 — FOOD ATTRIBUTE INPUTS

256 — LOCATION ATTRIBUTE INPUTS

258 — OPERATOR ATTRIBUTE INPUTS

260 — TARGET AGE RANGE

200

262 — HISTORICAL DATA

287 — PREDICTIVE ANALYTICS

285 — MODEL SELECTION / PARAMETER MODIFICATION / THRESHOLD SELECTION

270 — ANALYTICAL MODEL

272 — PRESENT OR PREDICTED AGE (E.G., QUALITY INDEX)

RECOMMENDATION OR CONTROL OUTPUT

FIG. 2

**FIG. 3**

400

405 — OBTAIN INFORMATION FROM SENSOR (E.G., NIR)

410 — OBTAIN MODEL PARAMETERS (E.G., OBTAIN COEFFICIENTS AND SPECIFIED INPUTS FOR USE CASE)

415 — APPLY MODEL TO DETERMINE OIL AGE

420 — TRIGGER ACTION

# FIG. 4

500

505 — OBTAIN INFORMATION FROM SENSOR (E.G., NIR)

510 — OBTAIN MODEL PARAMETERS (E.G., OBTAIN COEFFICIENTS AND SPECIFIED INPUTS FOR USE CASE)

515 — APPLY MODEL TO DETERMINE OIL AGE

520 — AUTOMATICALLY CONTROL EDIBLE OIL OPERATION (E.G., ONE OR MORE OF TOP-OFF, FILTRATION, REMOVAL, OR REPLACEMENT)

# FIG. 5

600

| 605 | OBTAIN INFORMATION FROM SENSOR (E.G., NIR) |
| 610 | OBTAIN MODEL PARAMETERS (E.G., OBTAIN COEFFICIENTS AND SPECIFIED INPUTS FOR USE CASE) |
| 615 | ON A RECURRING BASIS, AUTOMATICALLY TOP-OFF AND REMOVE SPECIFIED QUANTITIES OF OIL |
| 620 | APPLY MODEL TO DETERMINE OIL AGE |
| 625 | AUTOMATICALLY MODIFY CONTROL OF EDIBLE OIL OPERATION (E.G., ONE OR MORE OF TOP-OFF, FILTRATION, REMOVAL) |
| 630 | AUTOMATICALLY REPLACE OIL AT OR BEFORE PREDICTED END-OF-LIFE INDICATED BY EVALUATION OF MODEL |
| 635 | MANAGE REPLENISHMENT |

**FIG. 6**

700

705 — OBTAIN SURROGATE DATA FROM SENSOR

710 — DETERMINE SPECIFIED PARAMETER FROM SURROGATE DATA

715 — APPLY MODEL TO DETERMINE OIL AGE USING DETERMINED SPECIFIED PARAMETER FROM SURROGATE DATA

# FIG. 7

**FIG. 8**

915A
925A
935A

───── TPC ──── COLOR ─── FFA

t

**FIG. 9A**

915B
925B
935B

───── TPC ──── COLOR ──── FFA

t

**FIG. 9B**

v

945
965
955

t

**FIG. 9C**

FIG. 10A

FIG. 10B

EP 3 902 453 B1

FIG. 10C

FIG. 10D

EP 3 902 453 B1

**FIG. 11**

**EP 3 902 453 B1**

**Patent documents cited in the description**

- WO 2017002079 A1